# EUROPEAN PATENT SPECIFICATION

(11) **EP 3 466 381 B1**
(45) Date of publication and mention of the grant of the patent: **07.10.2020**
(21) Application number: 16903046.7
(22) Date of filing: 23.05.2016
(51) Int. Cl.: A61F 13/15, G05B 19/418

(54) **PRODUCTION METHOD FOR ABSORBENT ARTICLE**
HERSTELLUNGSVERFAHREN FÜR SAUGFÄHIGEN ARTIKEL
PROCÉDÉ DE PRODUCTION D'ARTICLE ABSORBANT

(43) Date of publication of application: 10.04.2019
(73) Proprietor: Unicharm Corporation, Shikokuchuo-shi, Ehime 799-0111 (JP)
(72) Inventor: HAGITA, Hiromi, Kanonji-shi Kagawa 769-1602 (JP)
(74) Representative: Staeger & Sperling Partnerschaftsgesellschaft mbB
(86) International application number: PCT/JP2016/065168
(87) International publication number: WO 2017/203564

(56) References cited:
- EP-A1- 2 612 632
- WO-A1-2009/031359
- JP-A- 2003 345 414
- JP-A- 2006 146 876
- JP-A- 2011 172 666
- JP-A- 2012 519 565
- JP-A- 2014 033 799
- JP-A- 2016 018 241
- US-A1- 2013 296 149

## Description

### Technical Field

The present invention relates to a method for manufacturing an absorbent article such as a disposable diaper.

### Background Art

Heretofore, a disposable diaper 1' has been known as an example of an absorbent article.

### Citation List

### Patent Literature

Patent Literature 1: Japanese Translation of PCT International Application Publication No. JP-T-2007-515218. Further prior art in this technical field is disclosed in documents US 2013/296149 A1 and EP 2 612 632 A1.

### Summary of Invention

### Technical Problem

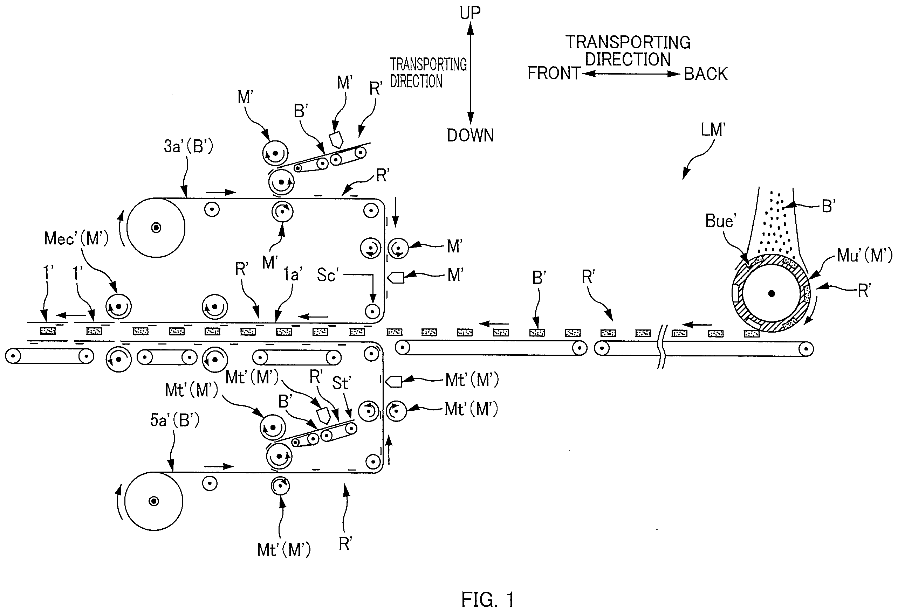

Fig. 1 is a schematic side view of a manufacturing line LM' for manufacturing disposable diapers 1' according to a reference example. In this manufacturing line LM', a plurality of components B', B', ... including a plurality of continuous sheets 3a' and 5a' are processed while being transported in a plurality of transport paths R', R', .., to thereby generate the diapers 1'. Moreover, in the line LM', a merge process for all the continuous sheets 3a' and 5a' of the plurality of continuous sheets 3a' and 5a' is completed at a predetermined merge completion position Sc' to generate a single composite sheet 1a', which is continuous in a transporting direction. The composite sheet 1a' is then cut at intervals in the transporting direction with an end-cutter device Mec' located at a transporting-direction downstream end, to thereby generate the diapers 1'.

Here, if any problem occurs at a certain position St', which is located upstream of the merge completion position Sc' in the manufacturing line LM', production of the line LM' is suspended. To be more specific, transport in each of the plurality of transport paths R', R', ... is suspended. After the problem is corrected, transport in each of the transport paths R', R', ... is restarted to restore the manufacturing line LM' to a producing state. However, in the case where processing devices Mt', Mt', ... located between the certain position St', where the problem has occurred, and the merge completion position Sc' are still in a processing state at the time of restart, this may cause a secondary trouble at the processing devices Mt', Mt', ... due to the processing devices Mt', Mt', ... being in the processing state. To prevent such a situation, it is considered preferable that at least some of the processing devices Mt', Mt', ... are brought into a standby state to then restart the transport in the state where the processing devices Mt', Mt', ... are in the standby state.

Meanwhile, during the suspension of the transport, dust and the like may attach to the components B', B' ... in the transport paths R', R', ..., which may consequently make the components B', B', ... unclean. To prevent this, it is conceivable to dispose of the components B', B' ... located in the transport paths R', R', ... during the suspension. Details are as follows.

First, a plurality of parts to compose the diapers 1' are present between each of processing devices M', M', ..., which are located transporting-direction upstream of the merge completion position Sc', and the merge completion position Sc', and the processing device M' at the position where the number of parts to compose the diapers 1' (including the number of parts in the middle of process with the processing device M') is the greatest among the processing devices M', M', ... is defined as an "uppermost-stream processing device Mu'".

With such a definition, focusing on an uppermost-stream part Bue' positioned in the uppermost-stream processing device Mu during the suspension of the transport among parts that can be associated with the diapers 1' among the plurality of components B', B', ...., it is conceivable to dispose of, as defective pieces, the diapers 1', 1', ... that pass the end-cutter device Mec' before the uppermost-stream part Bue' passes the position of the end-cutter device Mec' after the restart of the transport. In this way, it is possible to basically prevent the diapers 1' that are made unclean due to dust and the like as described above from being brought into the market.

However, here, suppose a case where the timing of switching the processing devices Mt' from the standby state to the processing state after the restart of the transport is significantly delayed. In this case, the defective diapers 1' to be disposed may increase by the number corresponding to the delay.

For example, in a case that, after the restart of the transport, the uppermost-stream part Bue' reaches the merge completion position Sc' and thereafter the part for which processing by the processing device Mt' is restarted passes the merge completion position Sc', the defective diapers 1' 1', ... to be disposed of increase by the number corresponding to the delay in the passing. This may consequently reduce the yields.

The present invention has been made in view of the above problems in known technique, and an advantage thereof is to make it possible to prevent an increase in the number of defective diapers to be disposed of due to delay of restoration of a processing device brought into a standby state as a result of suspension of transport to a processing state, and also to make it easier to prevent any trouble that may occur as a result of restart of the transport.

### Solution to Problem

A primary aspect of the invention for achieving the aforementioned objective is a method for preventing an increase in the number of defective absorbent articles in a method for manufacturing the absorbent article by processing a plurality of components including a plurality of continuous sheets while transporting the plurality of components in a plurality of transport paths, the method including:
a generation step of generating a single composite sheet that is continuous in a transporting direction by completing in a predetermined merge completion position a merge process of all the continuous sheets of the plurality of continuous sheets;
a generation step of generating the absorbent article by cutting the composite sheet at an interval in the transporting direction;
a transport suspension step of suspending transport in the plurality of transport paths at an occurrence of a problem in a certain position in the plurality of transport paths;
a standby step of bringing into a standby state at least some of processing devices among a plurality of processing devices located between the certain position where the problem has occurred and the merge completion position;
a transport restart step of restarting the transport, after correcting the problem, while some of the processing devices are maintained in the standby state; and
a restoration step of restoring to a processing state some of the processing devices in the standby state, after restarting the transport, wherein
in a case of defining, as an uppermost-stream processing device, a processing device in a position with a greatest number of parts to compose the absorbent article, among all processing devices located upstream of the merge completion position in the transporting direction, the parts being placed between a position of each processing device and the merge completion position and including the parts in the middle of being processed with the processing device,
the restoration step is performed such that a part for which processing is restarted in the restoration step passes the merge completion position before an uppermost-stream part reaches the merge completion position after the restart of the transport, the uppermost-stream part being a part that can be associated with the absorbent article among parts of the plurality of components, the uppermost-stream part being located in the uppermost-stream processing device during suspension of the transport.

Other features of the present invention will become apparent from the present description and the attached drawings.

### Advantageous Effects of Invention

According to the present invention, it is possible to prevent an increase in the number of defective diapers to be disposed of due to delay of restoration of a processing device brought into a standby state as a result of suspension of transport to a processing state, and also to make it easier to prevent any trouble that may occur as a result of restart of the transport.

### Brief Description of Drawings

[Fig. 1] Fig. 1 is a schematic side view of a manufacturing line LM' for manufacturing disposable diapers 1' according to a reference example.
[Figs. 2A and 2B] Fig. 2A is a schematic plan view illustrating an example of a disposable diaper 1 in a spread-out manner, and Fig. 2B is a cross-sectional view taken along B-B in Fig. 2A.
[Fig. 3] Fig. 3 is a schematic plan view illustrating a process for generating diapers 1.
[Fig. 4] Fig. 4 is a schematic side view of a manufacturing line LM of diapers 1 according to a present embodiment.
[Fig. 5] Fig. 5 is a schematic side view illustrating a back-sheet transport path R5a in an enlarged manner.
[Fig. 6] Fig. 6 is a schematic side view illustrating a top-sheet transport path R3a in an enlarged manner.
[Fig. 7] Fig. 7 is a schematic side view illustrating a base-sheet transport path R1a in an enlarged manner.

### Description of Embodiments

At least the following matters are made clear from the present description and the attached drawings.

Disclosed is a method for manufacturing an absorbent article by processing a plurality of components including a plurality of continuous sheets while transporting the plurality of components in a plurality of transport paths, the method for manufacturing including:
a generation step of generating a single composite sheet that is continuous in a transporting direction by completing in a predetermined merge completion position a merge process of all the continuous sheets of the plurality of continuous sheets;
a generation step of generating the absorbent article by cutting the composite sheet at an interval in the transporting direction;
a transport suspension step of suspending transport in the plurality of transport paths at an occurrence of a problem in a certain position in the plurality of transport paths;
a standby step of bringing into a standby state at least some of processing devices among a plurality of processing devices located between the certain position where the problem has occurred and the merge completion position;
a transport restart step of restarting the transport, after correcting the problem, while some of the processing devices are maintained in the standby state; and
a restoration step of restoring to a processing state some of the processing devices in the standby state, after restarting the transport, wherein
in a case of defining, as an uppermost-stream processing device, a processing device in a position with a greatest number of parts to compose the absorbent article, among all processing devices located upstream of the merge completion position in the transporting direction, the parts being placed between a position of each processing device and the merge completion position and including the parts in the middle of being processed with the processing device,
the restoration step is performed such that a part for which processing is restarted in the restoration step passes the merge completion position before an uppermost-stream part reaches the merge completion position after the restart of the transport, the uppermost-stream part being a part that can be associated with the absorbent article among parts of the plurality of components, the uppermost-stream part being located in the uppermost-stream processing device during suspension of the transport.

According to such a method for manufacturing an absorbent article, the restoration step is performed such that a part for which processing is restarted in the restoration step passes the merge completion position before the uppermost-stream part located at the uppermost-stream processing device at the time of the restart of the transport reaches the merge completion position. Hence, it is possible to prevent an increase in the number of defective absorbent articles due to a delay in restoration from the standby state to the processing state.

In addition, the processing device in the standby state is restored to the processing state after the restart of the transport. Hence, it is possible to prevent a trouble that may occur in a case of restarting the transport in the processing state and is consequently easier to prevent a trouble that may occur as a result of the restart of the transport.

In the method for manufacturing an absorbent article, it is preferable that some of the processing devices in the standby state include a first processing device and a second processing device located downstream of the first processing device in the transporting direction, and that the second processing device is restored to the processing state after the first processing device is restored to the processing state.

According to such a method for manufacturing an absorbent article, the second processing device can be restored to the processing state in sufficient time compared to a case that the first processing device and the second processing device are restored to the processing state at the same time. Hence, it is possible to perform stable restoration of the second processing device to the processing state.

Moreover, even in a case that the first processing device and the second processing device have a dependency relationship in which a problem occurs in a case where a part processed with the first processing device is not processed with the second processing device, it is possible to address the situation without any big problem as long as the processing devices are restored in the above-described order.

Furthermore, in a case that the first processing device located upstream is restored later than the second processing device located downstream, the number of defective absorbent articles may increase. However, it is possible to prevent such an increase as long as the processing devices are restored in the above-described order.

In the method for manufacturing an absorbent article, it is preferable that a dependency relationship in which a problem occurs if the second processing device does not process a part processed with the first processing device exists between the first processing device and the second processing device.

According to such a method for manufacturing an absorbent article, since the second processing device is restored to the processing state after the first processing device is restored to the processing state, it is possible to efficiently prevent occurrence of the above-described problem.

In the method for manufacturing an absorbent article, it is preferable that a control device configured to control the first processing device and the second processing device is provided, and that the control device includes an interlock for regulating a situation that the second processing device is restored to the processing state before the first processing device is restored to the processing state.

According to such a method for manufacturing an absorbent article, since the control device includes the above-described interlock, it is possible to efficiently prevent the second processing device located downstream from being restored to the processing state before the first processing device located upstream is restored to the processing state.

In the method for manufacturing an absorbent article, it is preferable that the composite sheet is transported on a downstream side of the merge completion position in the transporting direction while the composite sheet is in a state of being stretched in the transporting direction, and that the composite sheet is maintained in the state of being stretched in the transporting direction even during the suspension of the transport in the plurality of transport paths.

According to such a manufacturing method of an absorbent article, the composite sheet located downstream of the merge completion position is maintained in a state of being stretched in the transporting direction even during suspension of the transport as during the transport. Hence, it is possible to smoothly restart the transport downstream of the merge completion position. Consequently, it is also possible to smoothly restart the transport in each of the transport paths located to the upstream side of the merge completion position.

In the method for manufacturing an absorbent article, it is preferable that a conveyer configured to transport the composite sheet is provided, that the conveyer is a suction belt conveyer with a suction-holding function in an outer circumferential surface of an endless belt, and that the composite sheet is maintained in the state of being stretched in the transporting direction even during the suspension of the transport by being sucked and held to the outer circumferential surface of the endless belt.

According to such a manufacturing method of an absorbent article, it is possible to maintain the composite sheet in a state of being stretched in the transporting direction even during suspension of the transport.

In the method for manufacturing an absorbent article, it is preferable that a third processing device configured to process the composite sheet is provided in a transport path for transporting the composite sheet, that the third processing device includes a pair of rolls configured to rotate in a transporting direction of the composite sheet, the pair of rolls being configured to be switchable between a processing state with a small gap between the rolls and a standby state with a greater gap than the gap in the processing state, that in the processing state, the pair of rolls clamps the composite sheet to process the composite sheet, and that the third processing device is maintained in the processing state even during the suspension of the transport in the plurality of transport paths.

According to such a manufacturing method of an absorbent article, the third processing device is maintained in the processing state even during suspension of the transport. Hence, the pair of rolls of the third processing device is maintained in a state of clamping the composite sheet even during the suspension of the transport. This also effectively contributes to maintaining the composite sheet in a state of being stretched in the transporting direction.

In the method for manufacturing an absorbent article, it is preferable that a fourth processing device located in a position outside an area from the certain position where the problem has occurred to the merge completion position is provided, the fourth processing device is in a standby state at time of the restart of the transport, and that the fourth processing device is restored to the processing state such that a part for which processing is restarted after the fourth processing device is restored from the standby state to the processing state passes the merge completion position, before the uppermost-stream part reaches the merge completion position, after the restart of the transport.

According to such a method for manufacturing an absorbent article, the fourth processing device is restored to the processing state such that a part for which processing is restarted in the fourth processing device passes the merge completion position before the uppermost-stream part reaches the merge completion position at the time of the restart of the transport. Hence, it is possible to prevent an increase in the number of defective absorbent articles due to a delay in restoration of the fourth processing device in the standby state to the processing state.

### Present Embodiment

A method for manufacturing an absorbent article according to a present embodiment is employed in a manufacturing line LM of disposable diapers 1. In other words, the manufacturing method according to the present embodiment is used for manufacturing the disposable diapers 1 as an example of absorbent articles.

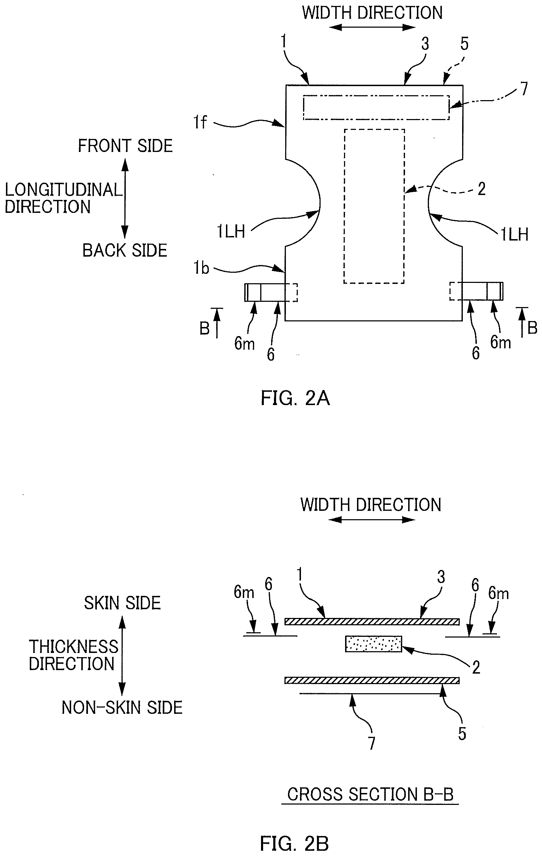

Fig. 2A is a schematic plan view illustrating an example of a disposable diaper 1 in a spread-out manner. Fig. 2B is a cross-sectional view taken along B-B in Fig. 2A.

This diaper 1 is a so-called tape-type diaper 1. Specifically, the diaper 1 is a type of diaper to be put on by a wearer by using fastening tapes 6 and a target tape 7. In a state where the diaper 1 is spread out by disengaging the fastening tapes 6 and the target tape 7 as in Fig. 2A, the diaper 1 has three directions perpendicular to one another, i.e., a longitudinal direction, a width direction, and a thickness direction.

The diaper 1 includes: an absorbent body 2 that absorbs excrement, such as urine; a liquid-permeable top sheet 3 that is arranged on a thickness-direction skin side of the absorbent body 2; and a liquid-impermeable back sheet 5 that is arranged on a non-skin side of the absorbent body 2 to prevent leakage of excrement to the non-skin side.

In the spread-out state in Fig. 2A, the top sheet 3 and the back sheet 5 have the same shape, which is a substantially hourglass shape when viewed from above. The portion narrowed inward in a width-direction in the substantially hourglass shape when viewed from above, functions as leg openings 1LH and 1LH when the diaper 1 is worn.

When the diaper 1 is worn, a portion 1f, of the spread-out diaper 1, on one longitudinal-direction side and a portion 1b of the spread-out diaper 1, on the other longitudinal-direction side, function respectively as a front-side portion 1f covering the ventral side of the wearer and a back-side portion 1b covering the back side of the wearer. To be able to keep the shape of the diaper 1 in such worn state, a pair of fastening tapes 6 and 6 each including a male material 6m of a hook-and-loop fastener is provided to the width-direction sides of the back-side portion 1b to protrude from the width-direction sides, and the target tape 7 is provided to a non-skin side surface of the front-side portion 1f, the target tape 7 being made of a female material of a hook-and-loop fastener, nonwoven fabric, or the like to enable engagement of the male materials 6m when the diaper 1 is worn

The absorbent body 2 includes an absorbent core made by forming a liquid-absorbent material into a substantially rectangular shape when viewed from above. Examples of the liquid-absorbent material include pulp fiber and superabsorbent polymer (SAP), and such materials are used here.

An example of a material of the top sheet 3 is nonwoven fabric containing thermoplastic resin fiber, such as polyethylene or polypropylene. Meanwhile, an example of a material of the back sheet 5 is a thermoplastic resin film, such as polyethylene. The materials, however, are not limited to these. For example, any material having liquid permeability and stretchability can be used as the material of the top sheet 3, and any material having liquid impermeability and stretchability can be used as the material of the back sheet.

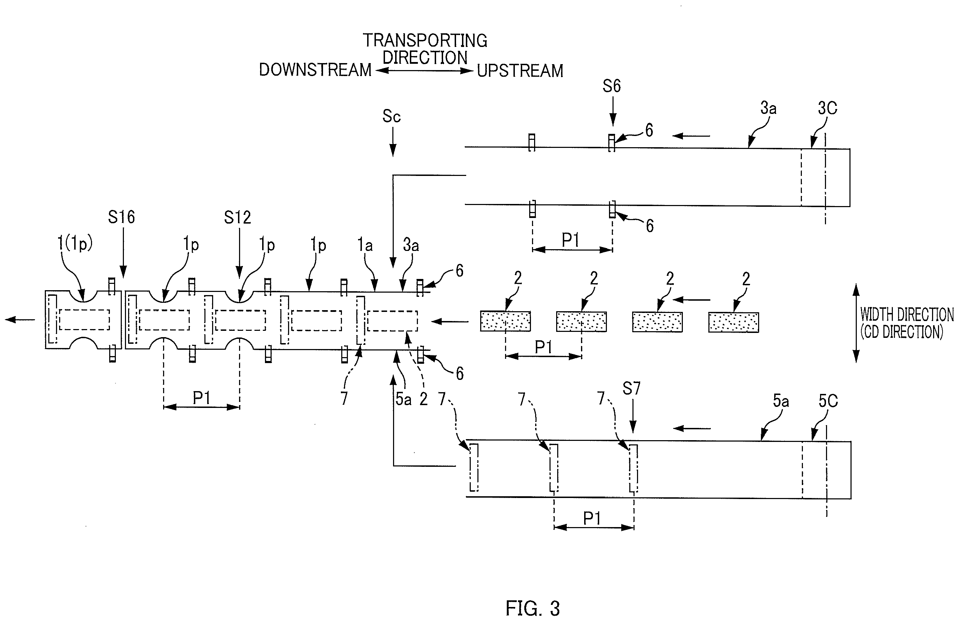

Fig. 3 is a schematic plan view illustrating a process for generating the diapers 1.

The top sheet 3 and the back sheet 5 are brought into the manufacturing line LM in the form of material coils 3C and 5C made by continuous sheets 3a and 5a, which are materials, wound in coils.

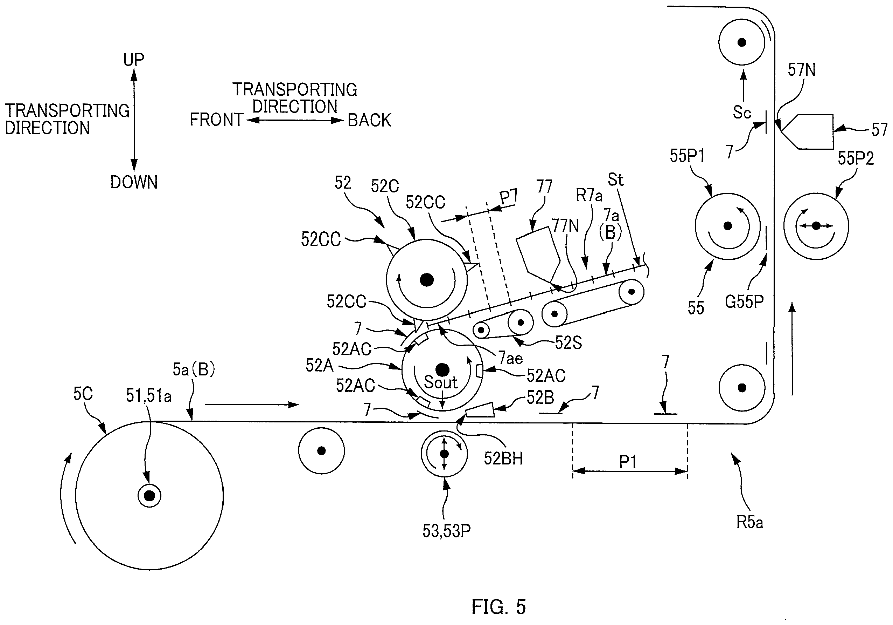

First, the back-sheet continuous sheet 5a fed from the material coil 5C is transported in the continuous direction as the transporting direction. During the transport, the single-sheet target tape 7 is fixed to a non-skin-side surface of the continuous sheet 5a at a predetermined transporting-direction position S7 intermittently with a product pitch P1 in the transporting direction with adhesive. The continuous sheet 5a is transported to a merge position Sc to merge with the top-sheet continuous sheet 3a. Note that the single-sheet target tape 7 is generated by cutting out a transporting-direction downstream-end portion 7ae of the target-tape continuous body 7a (cf. Fig. 5) transported in the continuous direction as the transporting direction.

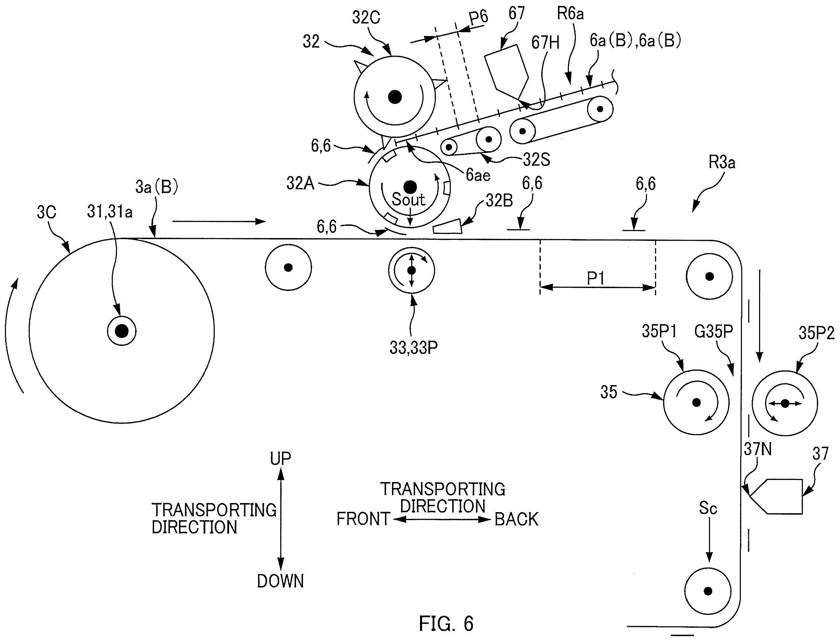

Similarly, the top-sheet continuous sheet 3a fed from the other material coil 3C is transported in the continuous direction as the transporting direction. During the transport, the width-direction pair of single-sheet fastening tapes 6 and 6 is fixed to a non-skin-side surface of the continuous sheet 3a at a predetermined transporting-direction position S6 intermittently with the product pitch P1 in the transporting direction with adhesive. The continuous sheet 3a is also transported to the above-described merge position Sc. Note that the pair of single-sheet fastening tapes 6 and 6 is also generated by cutting out a transporting-direction downstream-end portion 6ae of the pair of fastening tape continuous bodies 6a and 6a (cf. Fig. 6) transported in the continuous direction as the transporting direction.

Meanwhile, as illustrated in Fig. 3, each absorbent body 2 is generated by forming, in a single sheet, pulp fiber and SAP that are mixed with each other. The absorbent bodies 2 lined with the product pitch P1 in the transporting direction are each then transported to the above-described merge position Sc.

At the merge position Sc (corresponding to the merge completion position), the back-sheet continuous sheet 5a merges with the absorbent body 2 from the non-skin side of the absorbent body 2, and the top-sheet continuous sheet 3a merges with the absorbent body 2 from the skin side of the absorbent body 2, to join these three elements with adhesive in an integrated manner and thereby generate a diaper base sheet 1a (corresponding to the composite sheet). In other words, the diaper base sheet 1a is in a state where parts 1p each to be the diaper 1 at a final stage are continuously lined with the product pitch P1 in the transporting direction.

The diaper base sheet 1a is continuously transported in the transporting direction. At a predetermined position S12 in the transporting-direction, width-direction end portions of the base sheet 1a are cut out with the product pitch P1 in the transporting direction, to form each part 1p to be a diaper in the above-described substantially hourglass shape when viewed from above. Furthermore, at a predetermined position S16 located downstream of the predetermined position S12, the base sheet 1a is cut with the product pitch P1 in the transporting direction. In this way, a lowermost-stream part 1p of the parts 1p to be diapers is cut apart from the base sheet 1a, to consequently generate the diaper 1.

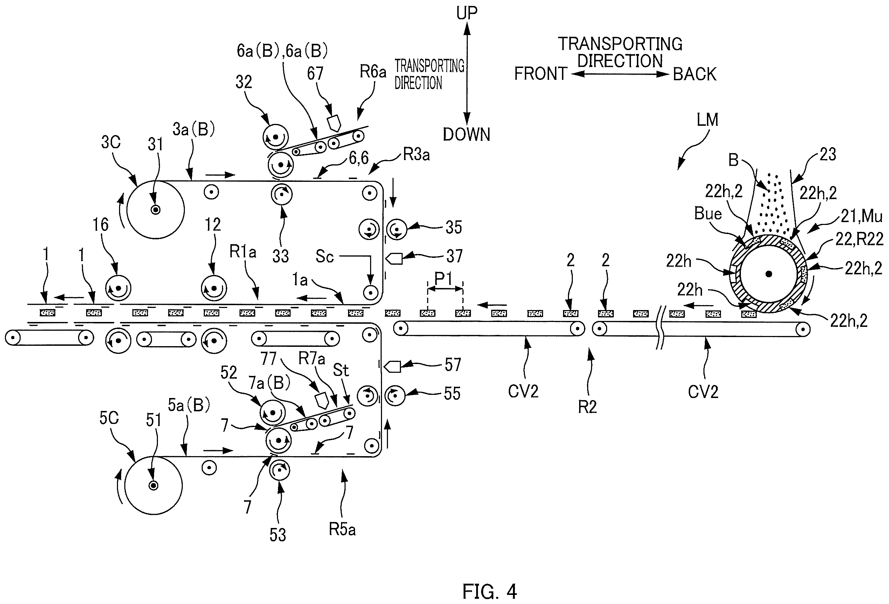

Fig. 4 is a schematic side view of the manufacturing line LM of the diapers 1.

The manufacturing line LM includes a plurality of transport paths R5a, R3a, R2, .... Specifically, the line IM includes the back-sheet transport path R5a for transporting the back-sheet continuous sheet 5a, the top-sheet transport path R3a for transporting the top-sheet continuous sheet 3a, and the absorbent-body transport path R2 for transporting the absorbent bodies 2. These three transport paths R5a, R3a, and R2 merge with each other at the above-described merge position Sc. At the transporting-direction downstream of the merge position Sc, a base-sheet transport path R1a for transporting the base sheet 1a of the diapers 1 is provided.

Furthermore, the line LM includes a target-tape transport path R6a for transporting the target-tape continuous body 7a and also a fastening-tape transport path R6a for transporting the fastening-tape continuous body 6a. The former target-tape transport path R7a is connected to the back-sheet transport path R5a via a slip-cutting device 52 to be described later, and the latter fastening-tape transport path R6a is connected to the top-sheet transport path R3a via a slip-cutting device 32 to be described later.

Each of the transport paths R5a, R3a, R2, R1a, R6a, and R7a includes an appropriate transport device, such as a belt conveyer and/or a transport roller. Unless otherwise described, a transport target, such as the continuous sheet 5a or 3a, is assumed to be transported in the transporting direction by the corresponding transport device in each of the transport paths R5a, R3a, R2, .... Examples of the belt conveyer include a general belt conveyer including, as a transport surface, an endless belt that is driven to rotate, and a suction belt conveyer having a suction function on an outer circumferential surface of an endless belt.

At least one or more processing devices for performing appropriate processing to generate the diapers 1 are provided to each of the transport paths R5a, R3a, R2, R1a, R6a, and R7a. Each of the processing devices performs a corresponding processing operation to generate the diapers 1. The "processing" here is processing that leaves a trace on each non-defective diaper 1 in a final state. Hence, for example, a so-called material joining process, which is not illustrated in this example, for joining materials, such as the continuous sheet 5a or 3a, to continuously supply the material without any shortage, leaves no trace of processing on non-defective diapers, and thus this process is not included in the "processing" here. Similarly, each material joining device that performs the material joining process is not included in the "processing device" here.

Furthermore, in this manufacturing line LM, a CD direction (in Fig. 4, a direction of penetrating the paper surface) is set as a width direction of the line IM. Although the CD direction corresponds to the horizontal direction in this example, the CD direction is not limited to this. Moreover, in this example, a vertical, up-down direction and a horizontal, front-back direction are set as two directions orthogonal to the CD direction, and each of the transporting directions of the back-sheet and top-sheet continuous sheets 5a and 3a and the transporting direction of the base sheet 1a corresponds to a direction defined by both the up-down direction and the front-back direction according to the position in the transporting direction. Each of the width directions of the back-sheet and top-sheet continuous sheets 5a and 3a and the width direction of the base material sheet 1a is parallel with the CD direction. In a case that a direction orthogonal to the CD direction and the transporting directions is defined as a Z direction, the Z direction is parallel with each of the thickness directions of the back-sheet and top-sheet continuous sheets 5a and 3a and the thickness direction of the base material sheet 1a.

Hereinafter, a description will be given of each of the transport paths R2, R5a, R7a, R3a, R6a, and R1a.

### <<<Absorbent-body Transport Path R2>>>

As illustrated in Fig. 4, a fiber-stacking drum device 21 configured to generate the absorbent body 2 is provided to the absorbent-body transport path R2, as a kind of a processing device for performing processing defined above. The device 21 includes a rotating drum 22 that is driven by a servomotor to rotate about a rotational shaft along the CD direction. On an outer circumferential surface of the rotating drum 22, a plurality of recessed portions 22h, 22h, ... are formed with the product pitch P1 in the direction of rotation. At a predetermined position in the direction of rotation, a spray duct 23 configured to spray pulp fiber and SAP (corresponding to a component) on the outer circumferential surface is provided. At the time when each recessed portion 22h passes the position of the spray duct 23, the pulp fiber and the SAP are laminated onto the recessed part 22h by suction through a suction hole (not illustrated) in a bottom surface of the recessed portion 22h, to thereby generate the absorbent body 2 in the recessed portion 22h.

Each recessed portion 22h faces a belt conveyer CV2 forming the absorbent-body transport path R2, at a predetermined position in the rotation direction. With this, at the time when each recessed portion 22h passes the above-described predetermined position, the absorbent body 2 is handed over from the recessed portion 22h to a transport surface of the belt conveyer CV2. The absorbent bodies 2 lined with the product pitch P1 in the transporting direction are then each transported to the above-described merge position Sc by the conveyer CV2.

Note that the suction operation via the suction hole can be enabled by connecting, to the suction hole, a blower (not illustrated) as a negative pressure source with an appropriate duct.

In this example, the spray duct 23 is configured to be switchable between a supply state of supplying pulp fiber and SAP and a non-supply state for not supplying these. The former supply state corresponds to a "processing state" of the fiber-stacking drum device 21, while the latter non-supply state corresponds to a "standby state" of the fiber-stacking drum device 21. In the standby state of the fiber-stacking drum device 21, the rotating drum 22 may rotate, and the suction hole may be in suction operation, in some cases.

Meanwhile, in the recessed portions 22h of the rotating drum 22, a process of generating the absorbent bodies 2 by lamination to the recessed portions 22h as described above is performed as processing performed on the pulp fiber and SAP. In this process, the pulp fiber and SAP are transported in the direction of rotation of the rotating drum 22 while being laminated to the recessed portions 22h. It can thus be understood that a transport path R22 for transporting the pulp fiber and SAP as a component B of the diapers 1 is also formed on the outer circumferential surface of the rotating drum 22. In this case, the rotation drum 22 functions as a transport device that transports the pulp fiber and SAP as the component B of the diapers 1. Hence, the "plurality of transport paths" described in the claims also include the above-described transport path R22 formed with the rotating drum 22.

### <<<Back-sheet Transport Path R5a>>>

Fig. 5 is a schematic side view illustrating the back-sheet transport path R5a in an enlarged manner.

In the back-sheet transport path R5a, a feeding device 51, a slip-cutting device 52, a temporary-pressing device 53, a main-pressing device 55, and an adhesive application device 57 lined in this order from the upstream to the downstream in the transporting direction are provided. Among the devices 51, 52, ..., each of the devices 52, 53, ... excluding the above-described feeding device 51, i.e., the slip-cutting device 52, the temporary-pressing device 53, the main-pressing device 55, and the adhesive application device 57, functions as a kind of a processing device for performing processing defined above.

The feeding device 51 includes a rotary driving shaft 51a along the CD direction. In a state where the material coil 5C of the back sheet 5 is installed to and supported with the rotary driving shaft 51a, the rotary driving shaft 51a is driven to rotate with a servomotor (not illustrated), to thereby feed the back-sheet continuous sheet 5a (corresponding to a component) from the material coil 5C.

The slip-cutting device 52 is a device configured to cut the target-tape continuous body 7a transported in the target-tape transport path R7a to generate the single-sheet target tapes 7 and that fixes the single-sheet target tapes 7 to the back-sheet continuous sheet 5a with the product pitch P1 in the transporting direction.

The device 52 includes: an anvil roll 52A that is driven with a servomotor (not illustrated) to rotate about a rotational shaft along the CD direction; a cutter roll 52C that faces an outer circumferential surface of the anvil roll 52A and is driven with a servomotor (not illustrated) to rotate about a rotational shaft along the CD direction; and a supply device 52S, such as a belt conveyer, that supplies the target-tape continuous body 7a to the anvil roll 52A.

Here, a plurality of suction holes (not illustrated) are formed in the outer circumferential surface of the anvil roll 52A, and the outer circumferential surface has suction force based on suction with the suction holes. The value (mpm) of the speed of feeding the target-tape continuous body 7a with the supply device 52S is set to be smaller than the value (mpm) of the circumferential speed of the anvil roll 52A. Hence, on the outer circumferential surface of the anvil roll 52A, a tip-side portion 7ae of the target-tape continuous body 7a is sucked and held in a surface contact state while being slid relative to the outer circumferential surface in a direction to be delayed compared to the outer circumferential direction.

Receiving blades 52AC, 52AC, ... that respectively receive a plurality of cutter blades 52CC, 52CC, ... on the outer circumferential surface of the cutter roll 52C are provided to the outer circumferential surface of the anvil roll 52A so as to face the respective cutter blades 52CC. At the time when each receiving blade 52AC passes the position of the cutter roll 52C, the corresponding cutter blade 52CC of the cutter roll 52C and the receiving blade 52AC sandwich the target-tape continuous body 7a on the outer circumferential surface to divide the continuous body 7a, to thereby cut out the tip-side portion 7ae. In this way, the single-sheet target tape 7 is generated.

The single-sheet target tape 7 cut out from the target-tape continuous body 7a is then sucked and held in a state of not being slid relative to the outer circumferential surface of the anvil roll 52A, to be transported in the direction of rotation at the value of the circumferential speed of the anvil roll 52A. Since the value of the circumferential speed is greater than the above-described value of the speed of feeding here, a gap is formed between the target tape 7 and the target tape 7 to be subsequently generated by dividing based on a difference of the speeds, to consequently allow the single-sheet target tapes 7 to be lined on the outer circumferential surface of the anvil roll 52A with the product pitch P1 in the direction of rotation.

At the time when each single-sheet target tape 7 sucked and held on the outer circumferential surface is transported in the direction of rotation and passes a position Sout (also referred to as a handover position Sout below) facing the back-sheet transport path R5a, the target tape 7 is handed over from the outer circumferential surface to the back-sheet continuous sheet 5a by joining using adhesive. Subsequently, the target tape 7 is transported in the transporting direction in a manner integrated with the back-sheet continuous sheet 5a.

The adhesive used for the joining is applied in advance when the target tapes 7 are still in the form of the continuous body 7a, and this will be described together with the target-tape transport path R7a.

At the position slightly downstream of the handover position Sout, a suction port 52BH of the suction blower 52B is provided. Assume a case where the target tape 7 is not handed over to the back-sheet continuous sheet 5a at the handover position Sout, the suction blower 52B operates to be able to suck and thereby collect the target tape 7 that is sucked and held on the outer circumferential surface of the anvil roll 52A with the suction port 52BH. This means that, in a state where the suction blower 52B is not in operation, the target tape 7 can basically be handed over to the back-sheet continuous sheet 5a. Hence, this state corresponds to the "processing state" of the slip-cutting device 52. In contrast, in a state where the suction blower 52B is in operation, the target tape 7 on the outer circumferential surface of the anvil roll 52A is sucked with the suction blower 52B and is thus not handed over to the back-sheet continuous sheet 5a. Hence, this state corresponds to the "standby state" of the slip-cutting device 52.

The temporary-pressing device 53 is a device configured to press the back-sheet continuous sheet 5a toward the outer circumferential surface of the anvil roll 52A of the slip-cutting device 52. With this, the target tape 7 is temporarily fixed to the back-sheet continuous sheet 5a with the above-described adhesive.

The device 53 includes a temporary-pressing roll 53P provided at the above-described handover position Sout to face the outer circumferential surface of the anvil roll 52A. The temporary-pressing roll 53P is driven by a servomotor (not illustrated) to rotate about a rotational shaft along the CD direction. The roll 53P is provided in a position to be able to sandwich the back-sheet continuous sheet 5a in the thickness direction with the outer circumferential surface of the anvil roll 52A, and is supported to be able to reciprocate in the direction of the rotation radius of the anvil roll 52A, with an actuator (not illustrated), such as a hydraulic cylinder.

When the temporary-pressing roll 53P is moved to a pressing position on the inner side of the direction of the rotation radius, the roll 53P enters the "processing state", which is a state of pressing the back-sheet continuous sheet 5a to the outer circumferential surface of the anvil roll 52A, to temporarily fix the target tape 7 on the outer circumferential surface of the anvil roll 52A to the back-sheet continuous sheet 5a. In contrast, when the temporary-pressing roll 53P is moved to a withdrawal position on the outer side of the direction of the rotation radius, the roll 53P enters the "standby state", which is a state of not pressing the back-sheet continuous sheet 5a to the outer circumferential surface of the anvil roll 52A, and thus not to temporarily fix the target tape 7 on the outer circumferential surface of the anvil roll 52A to the back-sheet continuous sheet 5a.

The main-pressing device 55 is a device configured to fix the temporarily fixed single-sheet target tape 7 to the back-sheet continuous sheet 5a. Specifically, the device 55 clamps together the back-sheet continuous sheet 5a and the target tape 7 temporarily fixed to the sheet 5a with adhesive, in the thickness direction with great clamping force, to fix the target tape 7 to the sheet 5a.

The device 55 includes a pair of rolls 55P1 and 55P2 that is driven by a servomotor (not illustrated) to rotate about a rotational shaft along the CD direction. The pair of rolls 55P1 and 55P2 is provided at positions where the pair of rolls 55P1 and 55P2 can sandwich the back-sheet continuous sheet 5a from both sides in the thickness direction of the back-sheet continuous sheet 5a. While the one roll 55P1 is rotatably supported in a fixed position, the other roll 55P2 is supported with an appropriate actuator (not illustrated), such as a hydraulic cylinder, in such a manner that the length of a gap G55P between the other roll 55P2 and the one roll 55P1 can be changed. By controlling the actuator, the device 55 can be switched between the "processing state", which is a state where the device clamps the back-sheet continuous sheet 5a with the target tape 7 temporarily fixed, and the "standby state", which is a state of not clamping the back-sheet continuous sheet 5a.

The adhesive application device 57 is a device that applies adhesive to the skin-side surface of the back-sheet continuous sheet 5a. The applied adhesive is used to join the absorbent bodies 2 and the top-sheet continuous sheet 3a to the continuous sheet 5a. The application device 57 includes: a nozzle 57N that discharges the adhesive toward the skin-side surface of the continuous sheet 5a; a pump (not illustrated) that supplies the adhesive to the nozzle 57N; and a valve (not illustrated) that is provided to a passage of the adhesive connecting the nozzle 57N and the pump. By opening and closing the valve, the device 57 can be switched between the "processing state", which is a state of applying the adhesive, and the "standby state", which is a state of not applying the adhesive. Examples of an application pattern of the adhesive can include an Ω pattern, a spiral pattern, a stripe pattern, and the like.

### <<<Target-tape Transport Path R7a>>>

As illustrated in Fig. 5, an adhesive application device 77 is provided to the target-tape transport path R7a, as a kind of a processing device configured to perform processing defined above. The adhesive application device 77 applies the above-described adhesive to the target-tape continuous body 7a (corresponding to a component). As already described, the adhesive is used to fix the single-sheet target tape 7 to the back-sheet continuous sheet 5a.

A configuration of the adhesive application device 77 is substantially the same as that of the above-described adhesive application device 57. Specifically, the application device 77, as the above-described application device 57, includes: a nozzle 77N that discharges adhesive toward the target-tape continuous body 7a; a pump (not illustrated) that supplies the adhesive to the nozzle 77N; and a valve (not illustrated) that is provided to a passage connecting the nozzle 77N and the pump. By opening and closing the valve, the device 77 can be switched between the "processing state", which is a state of applying the adhesive, and the "standby state", which is a state of not applying the adhesive. Hence, a further description is omitted.

### <<<Top-sheet Transport Path R3a>>>

Fig. 6 is a schematic side view illustrating the top-sheet transport path R3a in an enlarged manner.

In the top-sheet transport path R3a, as the back-sheet transport path R5a, a feeding device 31, a slip-cutting device 32, a temporary-pressing device 33, a main-pressing device 35, and an adhesive application device 37 lined in this order from the upstream to the downstream in the transporting direction are provided. Each of the devices 32, 33, ... excluding the above-described feeding device 31, among the devices 31, 32, ..., i.e., the slip-cutting device 32, the temporary-pressing device 33, the main-pressing device 35, and the adhesive application device 37, functions as a kind of a processing device configured to perform processing as defined above.

The feeding device 31 includes a rotary driving shaft 31a along the CD direction. In a state where the material coil 3C of the top sheet 3 is installed to and supported with the rotary driving shaft 31a, the rotary driving shaft 31a is driven to rotate with a servomotor (not illustrated), to thereby feed the top-sheet continuous sheet 3a (corresponding to a component) from the material coil 3C.

The slip-cutting device 32 is a device configured to cut the pair of fastening-tape continuous bodies 6a and 6a transported in the fastening-tape transport path R6a to generate a pair of single-sheet fastening tapes 6 and 6 and to fix the pair of single-sheet target tapes 6 and 6 to the top-sheet continuous sheet 3a with the product pitch P1 in the transporting direction with adhesive.

Note that a configuration of the device 32 is substantially the same as that of the slip-cutting device 52 in the back-sheet transport path R5a described above. Specifically, the device 32 also includes an anvil roll 32A, a cutter roll 32C, a supply device 32S, such as a belt conveyer, and a suction blower 32B. With this configuration, the device 32 performs a slip-cutting operation similar to that of the above-described device 52. By reading the description of the above slip-cutting device 52 while replacing the "target-tape continuous sheet 7a", the "single-sheet target tape 7", and the "back-sheet continuous sheet 5a" with the "pair of fastening tape continuous bodies 6a and 6a", the "pair of single-sheet fastening tapes 6 and 6", and the "top-sheet continuous sheet 3a", the description simply serves as a description of an operation and the like of the slip-cutting device 32. Hence, a further description is omitted.

The adhesive for joining the fastening tapes 6 and 6 to the top-sheet continuous sheet 3a is applied in advance when the fastening tapes 6 are still in the form of the continuous body 6a, and this will be described together with the fastening-tape transport path R6a.

The temporary-pressing device 33 is a device configured to press the top-sheet continuous sheet 3a toward the outer circumferential surface of the anvil roll 32A of the slip-cutting device 32. With this, the pair of fastening tapes 6 and 6 is temporarily fixed to the top-sheet continuous sheet 3a with the above-described adhesive.

Note that a configuration of the device 33 is substantially the same as that of the temporary-pressing device 53 in the back-sheet transport path R5a described above. Specifically, the device 33 also includes a temporary-pressing roll 33P and an actuator (not illustrated), such as a hydraulic cylinder, configured to cause the roll 33P to reciprocate in a rotation radius direction of the anvil roll 32A. With this, the device 33 performs a temporary-fixing operation similar to that of the above-described device 53 on the top-sheet continuous sheet 3a and the pair of fastening tapes 6 and 6. Specifically, by causing the temporary-pressing roll 33P to reciprocate in the direction of the rotation radius of the anvil roll 32A, the device 33 can be switched between the "processing state", which is a state of pressing the top-sheet continuous sheet 3a to the outer circumferential surface of the anvil roll 32A, and the "standby state", which is a state of not pressing the top-sheet continuous sheet 3a. Hence, a further description is omitted.

The main-pressing device 35 is a device configured to fix the pair of temporarily fixed single-sheet fastening tapes 6 and 6 to the top-sheet continuous sheet 3a. Specifically, the device 35 clamps together the top-sheet continuous sheet 3a and the pair of fastening tapes 6 and 6 temporarily fixed to the sheet 3a with adhesive, in the thickness direction with a great clamping force, to fix the pair of fastening tapes 6 and 6 to the sheet 3a.

Note that a configuration of the device 35 is substantially the same as that of the main-pressing device 55 in the back-sheet transport path R5a described above. Specifically, the device 35 also includes a pair of rolls 35P1 and 35P2 provided at positions where the pair of rolls 35P1 and 35P2 can sandwich the top-sheet continuous sheet 3a from both sides in the thickness direction of the top-sheet continuous sheet 3a. While the one roll 35P1 is rotatably supported at a fixed position, the other roll 35P2 is supported with an appropriate actuator (not illustrated), such as a hydraulic cylinder, in such a manner that the length of a gap G35P between the other roll 35P2 and the one roll 35P1 can be changed. With this, the device 35 performs a fixing operation similar to that of the above-described device 55, on the top-sheet continuous sheet 3a and the pair of fastening tapes 6 and 6. In other words, by controlling the actuator, the device 35 can be switched between the "processing state", which is a state of clamping the top-sheet continuous sheet 3a to which the pair of fastening tapes 6 and 6 is temporarily fixed, and the "standby state", which is a state of not clamping the top-sheet continuous sheet 3a. Hence, a further description is omitted.

The adhesive application device 37 is a device configured to apply adhesive to the non-skin-side surface of the top-sheet continuous sheet 3a. The applied adhesive is used to fix the absorbent bodies 2 and the back-sheet continuous sheet 5a to the continuous sheet 3a.

Note that a configuration of the device 37 is substantially the same as that of the adhesive application device 57 in the back-sheet transport path R5a described above. Specifically, the device 37 also includes: a nozzle 37N configured to discharge the adhesive toward the non-skin-side surface of the continuous sheet 3a; a pump (not illustrated) configured to supply the adhesive to the nozzle 37N; and a valve (not illustrated) that is provided to a passage of the adhesive connecting the nozzle 37N and the pump. By opening and closing the valve, the device 37 can be switched between the "processing state", which is a state of applying the adhesive, and the "standby state", which is a state of not applying the adhesive. Hence, a further description is omitted.

### <<<Fastening-tape Transport Path R6a>>>

An adhesive application device 67 is provided to the fastening-tape transport path R6a, as a kind of a processing device configured to perform processing defined above. This adhesive application device 67 applies the above-described adhesive to the pair of fastening-tape continuous bodies 6a and 6a (corresponding to a component). As already described, the adhesive is used to fix the pair of single-sheet fastening tapes 6 and 6 to the top-sheet continuous sheet 3a.

A configuration of the adhesive application device 67 is substantially the same as that of the above-described adhesive application device 37. The application device 67, as the above-described application device 37, includes: nozzles 67N configured to discharge adhesive toward the pair of fastening-tape continuous bodies 6a and 6a; a pump(s) (not illustrated) configured to supply the adhesive to the nozzles 67N; and a valve (s) (not illustrated) provided to a passage connecting the nozzles 67N and the pump. By opening and closing the valve(s), the device 67 can be switched between the "processing state", which is a state of applying the adhesive, and the "standby state", which is a state of not applying the adhesive. Hence, a further description is omitted.

### «<Base-sheet Transport Path R1a>>>

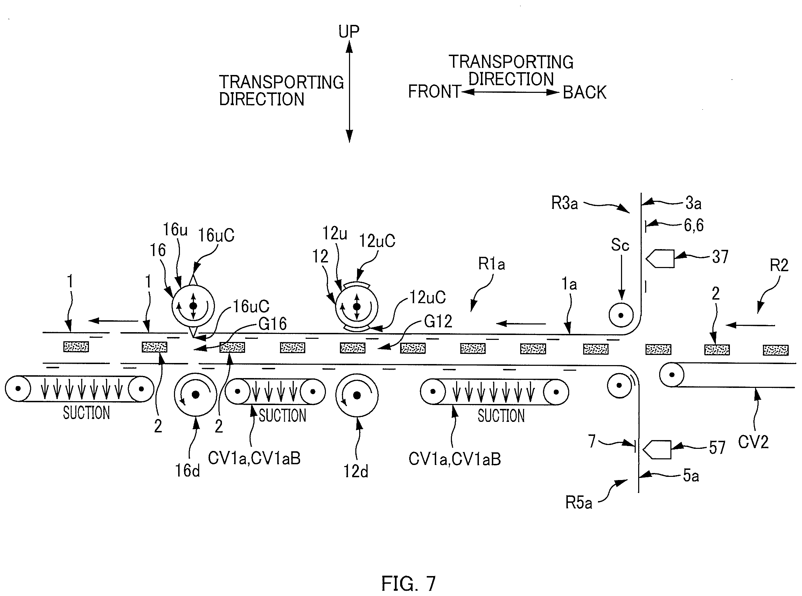

Fig. 7 is a schematic side view illustrating the base-sheet transport path R1a in an enlarged manner.

The base-sheet transport path R1a is located downstream of the merge position Sc. In the base-sheet transport path R1a, a leg-hole cutting device 12 and an end-cutting device 16 lined in this order from the upstream to the downstream in the transporting direction are provided, as a kind of a processing device configured to perform processing defined above. First, the former cutting device 12 clamps and cuts both sides in the CD-direction of the diaper base sheet 1a transported from the merge position Sc, to thereby form, in the base sheet 1a, a pair of leg openings 1LH and 1LH in the CD direction. Subsequently, the latter cutting device 16 cuts the base sheet 1a in a position between the absorbent bodies 2 and 2 adjacent to each other in the transporting direction, to cut out a downstream-end portion of the base sheet 1a by clamping and thereby generate the diaper 1 in Fig. 2A.

The leg-hole cutting device 12 includes a pair of upper and lower rolls 12u and 12d that is driven with a servomotor (not illustrated) to rotate about a rotational shaft along the CD direction. The upper roll 12u is a cutter roll. Specifically, cutter blades 12uC and 12uC are provided at a predetermined angle in the direction of rotation on the outer circumferential surface of the upper roll 12u, each of the cutter blades 12uC and 12uC having a curved shape corresponding to the shape of the leg opening 1LH of the diaper 1. Meanwhile, the lower roll 12d is an anvil roll configured to receive, on an outer circumferential surface of the lower roll 12d, the cutting blades 12uC. Rotation operations of the rolls 12u and 12d are basically performed together with the transport operation of the base sheet 1a. Specifically, when a pair of portions, of the base sheet 1a, where the leg openings 1LH are to be formed, i.e., portions located on CD-direction outer sides of the absorbent body 2 in the base sheet 1a, passes the position of the leg-hole cutting device 12, the upper roll 12u and the lower roll 12d rotate such that the corresponding cutter blades 12uC face the portions, to thereby form the leg openings 1LH and 1LH in the base sheet 1a.

In this example, while the lower roll 12d is rotatably supported in a fixed position, the upper roll 12u is supported by an appropriate actuator (not illustrated), such as a hydraulic cylinder, in such a manner that the length of a gap G12 between the upper roll 12a and the lower roll 12d can be changed. By controlling the actuator, it is possible to switch between a state of setting the gap G12 to be small to clamp the base sheet 1a with the upper and lower rolls 12u and 12d and cut out the leg openings 1LH and a state of setting the gap G12 to be large not to cut out the leg openings 1LH. The former state corresponds to a "processing state" of the leg-hole cutting device 12, while the latter state corresponds to a "standby state" of the leg-hole cutting device 12.

The end-cutting device 16 also includes a pair of upper and lower rolls 16u and 16d each driven with a servomotor (not illustrated) to rotate about a rotational shaft along the CD direction. The upper roll 16u is a cutter roll. Specifically, cutter blades 16uC and 16uC are provided at a predetermined angle in the direction of rotation on the outer circumferential surface of the upper roll 16u, each of the cutter blades 16uC and 16uC being in the form of a straight blade in the CD direction. Meanwhile, the lower roll 16d is an anvil roll configured to receive the cutting blades 16uC on an outer circumferential surface of the lower roll 16d. Rotation operations of the rolls 16u and 16d are basically performed together with the transport operation of the base sheet 1a. Specifically, when a portion of the base sheet 1a to be cut, i.e., a portion between the absorbent bodies 2 and 2 adjacent to each other in the transporting direction in the base sheet 1a, passes the position of the end-cutting device 16, the upper roll 16u and the lower roll 16d rotate such that the corresponding cutter blade 16uC faces the portion, to cut out the downstream-end portion from the base sheet 1a and thereby generate the diaper 1.

In this example, while the lower roll 16d is rotatably supported in a fixed position, the upper roll 16u is supported with an appropriate actuator (not illustrated), such as a hydraulic cylinder, in such a manner that the length of a gap G16 between the upper roll 16u and the lower roll 16d can be changed. By thus controlling the actuator, it is possible to switch between a state of setting the gap G16 to be small to clamp the base sheet 1a with the upper and lower rolls 16u and 16d and cut the base sheet 1a and a state of setting the gap G16 to be large not to cut the base sheet 1a. The former state corresponds to a "processing state" of the end-cutting device 16, while the latter state corresponds to a "standby state" of the end-cutting device 16.

The transport devices in the transport paths R1a, R2, R3a, R5a, R6a, and R7a and the above-described processing devices 12, 16, 21, 32, 33, 35, 37, 52, 53, 55, 57, 67, and 77 in the manufacturing line LM operate together on the basis of, for example, a synchronization signal. Here, the "synchronization signal" includes unit signals that are output repeatedly, and each unit signal is a rotational angle signal having a rotational angle value of, for example, 0 degrees to 360 degrees. The transport devices in the transport paths R1a, R2, ..., the processing devices 12, 16, ..., and the like each have a systematic unit operation to repeatedly perform for each corresponding part of the top-sheet and back-sheet continuous sheets 3a and 5a, the target-tape and fastening-tape continuous bodies 7a and 6a, and the base sheet 1a, the each corresponding part composing the diaper 1. Furthermore, each unit operation is basically associated with a unit signal in one-to-one correspondence through position control or speed control of the corresponding one of the above-described servomotors.

For example, for the transport device in each of the transport paths R1a, R2, R3a, and R5a, a transport operation of the product pitch P1 corresponding to the position of the transport device is associated as the unit operation. Meanwhile, for the transport device in each of the transport paths R6a and R7a, a transport operation of the product pitch P6 or P7 corresponding to the position of the transport device is associated as the unit operation. Furthermore, for each of the processing devices 12, 16, 21, 32, 33, 35, 52, 53, and 55, a rotation operation for the product pitch P1 corresponding to the position of the device is associated as the unit operation. Meanwhile, for each of the processing devices 37 and 57, an adhesive discharge operation for the product pitch P1 corresponding to the position of the device is associated as the unit operation. Furthermore, for each of the supply device 32S included in the processing device 32 and the supply device 52S included in the processing device 52, a supply operation for the product pitch P6 or P7 corresponding to the position of the device is associated as the unit operation. Further, for each of the processing devices 67 and 77, an adhesive discharge operation for the product pitch P6 or P7 corresponding to the position of the device is associated as the unit operation.

With this, basically, every time, the unit signal of the synchronization signal is output, each of the transport devices in the transport paths R1a, R2, R3a, and R5a performs the transport operation for the product pitch P1 corresponding to the position of the transport device, and each of the transport devices in the transport paths R6a and R7a performs the transport operation for the product pitch P6 or P7 corresponding to the position of the transport device. Further, each of the processing devices 12, 16, 21, 32, 33, 35, 52, 53, and 55 also performs the rotation operation for the product pitch P1 corresponding to the position of the device, and each of the processing devices 37 and 57 also performs the discharge operation for the product pitch P1 corresponding to the position of the device. Furthermore, each of the processing devices 32S and 52S also performs the supply operation for the product pitch P6 or P7 corresponding to the position of the device, and each of the processing devices 67 and 77 also performs the adhesive discharge operation for the product pitch P6 or P7 corresponding to the position of the device.

In a case that a problem has occurred in the manufacturing line LM, transport in each of the transport paths R1a, R2, R3a, R5a, R6a, and R7a is suspended. For example, upon detection, by an operator or an appropriate sensor, that a portion of the target-tape continuous body 7a is close to break in the target-tape transport path R7a, the transport in each of the transport paths R1a, R2, R3a, R5a, R6a, and R7a of the manufacturing line LM is suspended.

In addition, together with this suspension, all the operations related to transport are also suspended. For example, together with the suspension, the rotation operation of the rotating drum 22 of the fiber-stacking drum device 21, the rotation operations of the rotational shafts 31a and 51a of the feeding devices 31 and 51, the rotation operations of the anvil rolls 32A and 52A and the cutter rolls 32C and 52C and the supply operations of the supply devices 32S and 52S of the slip-cutting devices 32 and 52, the rotation operations of the temporary-pressing rolls 33P and 35P of the temporary-pressing devices 33 and 53, the rotation operations of the pair of rolls 35P1 and 35P2 and the pair of rolls 55P1 and 55P2 of the main-pressing devices 35 and 55, the rotation operations of the pair of upper and lower rolls 12u and 12d of the leg-hole cutting device 12, and the rotation operations of the pair of upper and lower rolls 16u and 16d of the end-cutting device 16, are also suspended. Consequently, the production in the manufacturing line LM is suspended.

During the suspension of transport, the operator, for example, reinforces the portion close to break in the target-tape continuous body 7a with an appropriate adhesive tape, to thereby correct the problem. After the correction of the problem, the transport in each of the transport paths R1a, R2, R3a, R5a, R6a, and R7a is restarted to restore the manufacturing line IM to a production state. At this event, not only the transport of each of the transport paths R1a, R2, R3a, R5a, R6a, and R7a, but also all the above-described operations related to the transport are restarted together. Specifically, the rotation operation of the rotating drum 22 of the fiber-stacking drum device 21, the rotation operations of the rotational shafts 31a and 51a of the feeding devices 31 and 51, the rotation operations of the anvil rolls 32A and 52A and the cutter rolls 32C and 52C and the supply operations of the supply devices 32S and 52S of the slip-cutting devices 32 and 52, the rotation operations of the temporary-pressing rolls 33P and 53P of the temporary-pressing devices 33 and 53, the rotation operations of the pair of rolls 35P1 and 35P2 and the pair of rolls 55P1 and 55P2 of the main-pressing devices 35 and 55, the rotation operations of the pair of upper and lower rolls 12u and 12d of the leg-hole cutting device 12, and the rotation operations of the pair of upper and lower rolls 16u and 16d of the end-cutting device 16 that are suspended together with the suspension of the transport, are restarted together.

However, if the processing devices 77, 52, 53, 55, and 57 located in an area from the position St where the problem has occurred, i.e., in this example, the position St of the cause due to which the target-tape continuous body 7a is close to break, to the merge position Sc (also referred to as a secondary-trouble risk area below) are maintained in the above-described "processing state" at the time of such restart of the transport, the processing devices 77, 52, 53, 55, and 57 may cause a secondary trouble due to being in the processing state since the processing devices 77, 52, 53, 55, and 57 are, in a sense, located immediately downstream of the position where the problem has occurred.

In view of this, in the present embodiment, each of the processing devices 77, 52, 53, 55, and 57 located in such a secondary-trouble risk area is brought into the above-described "standby state", and the transport in each of the transport paths R1a, R2, R3a, R5a, R6a, and R7a is restarted in the standby state. Specifically, in this example, the adhesive application device 77 of the target-tape transport path R7a and the slip-cutting device 52, the temporary-pressing device 53, the main-pressing device 55, and the adhesive application device 57 of the back-sheet transport path R5a are located as the processing devices in the area. Hence, first, each of devices 77, 52, 53, 55, and 57 is switched from the processing state to the standby state during or before the above-described suspension of the transport. After the correction of the problem, each of the devices 77, 52, 53, 55, and 57 restarts the transport while being in the standby state, and after the restart, each of the devices 77, 52, 53, 55, and 57 in the standby state is restored to the processing state.

In contrast, the processing devices 21, 32, 33, 35, 37, 67, 12, and 16 in the absorbent-body transport path R2, the top-sheet transport path R3a, the fastening-tape transport path R6a, and the base-sheet transport path R1a are located outside the secondary-trouble risk area. Hence, in this example, each of the processing devices 21, 32, 33, 35, 37, 67, 12, and 16 is in the processing state from the time of the restart of the transport. For example, although the fiber-stacking drum device 21 in the absorbent-body transport path R2, the adhesive application device 37 in the top-sheet transport path R3a, and the adhesive application device 67 in the fastening-tape transport path R6a are brought into the standby state once during the suspension of the transport, the fiber-stacking drum device 21 is already restored to the processing state of supplying pulp fiber and SAP from the spray duct 23, and each of the adhesive application devices 37 and 67 is already restored to the processing state of discharging the adhesive from the nozzle 37N or 67N, before the start, such as immediately before the start of the transport. Hence, each of the devices 21, 37, and 67 is already in the processing state at the time of the restart of the transport. Each of the slip-cutting device 32, the temporary-pressing device 33, and the main-pressing device 35 in the top-sheet transport path R3a and the leg-hole cutting device 12 and the end-cutting device 16 in the base-sheet transport path R1a is maintained in the processing state without being switched to the standby state even during the suspension of the transport. Hence, each of the devices 32, 33, 35, 12, and 16 is already in the processing state at the time of the restart of the transport.

Here, each of the above-described devices 77, 52, 53, 55, and 57 maintained in the standby state at the time of the restart of the transport needs to be restored to the processing state at an appropriate timing after the restart of the transport. The restoring timing for restoring each of the devices 77, 52, 53, 55, and 57 to the processing state may be determined on the basis of the following view.

First as illustrated in Fig. 4, during the suspension of the transport, dust or the like may attach to the components B, B..., such as the continuous sheets 3a and 4a, the continuous bodies 6a and 7a, and the absorbent bodies 2 in the transport paths R1a, R2, R3a, R5a, R6a, and R7a, such that the components B, B, ... may become unclean. From the viewpoint of preventing the defective diapers 1 that have become unclean from being brought to the market, it is conceivable to dispose of the components B, B ... located in the transport paths R1a, R2, R3a, R5a, R6a, and R7a during the suspension. Such disposal may be performed as follows, for example.

As illustrated in Fig. 4, between each of the processing devices 21, 37, 35, 33, 32, 67, 57, 55, 53, 52, and 77 located upstream of the merge position Sc in the transporting direction and the merge position Sc, a plurality of parts to compose the diapers 1 are lined in the transporting direction.

For example, as illustrated in Fig. 4, between the fiber-stacking drum device 21 in the absorbent-body transport path R2 and the merge position Sc, a plurality of absorbent bodies 2 lined in the transporting direction including the absorbent bodies 2 in the middle of being generated with the rotating drum 22 exist as the parts to compose the diapers 1. As illustrated in Fig. 5, between the slip-cutting device 52 in the back-sheet transport path R5a and the merge position Sc, a plurality of parts to compose the diapers 1 lined in the transporting direction with the product pitch P1 exist on the back-sheet continuous sheet 5a; further, between the adhesive application device 77 in the target-tape transport path R7a connected in the upstream through the slip-cutting device 52 and the merge position Sc, the plurality of single-sheet target tapes 7 located on the anvil roll 52A of the slip-cutting device 52 and lined in the transporting direction further exist as the parts to compose the diapers 1, in addition to the parts to compose the diapers 1 on the continuous sheet 5a, and a plurality of parts to compose the diapers 1 lined in the transporting direction with the product pitch P7 also exist on the target-tape continuous body 7a. Similarly, as illustrated in Fig. 6, between the slip-cutting device 32 in the top-sheet transport path R3a and the merge position Sc, a plurality of parts to compose the diapers 1 lined in the transporting direction with the product pitch P1 exist on the top-sheet continuous sheet 3a; further, between the adhesive application device 67 in the fastening-tape transport path R6a connected in the upstream through the slip-cutting device 32 and the merge position Sc, the plurality of pairs of single-sheet fastening tapes 6 and 6 in the CD direction located on the anvil roll 32A of the slip-cutting device 32 and lined in the transporting direction further exist as the parts to compose the diapers 1, in addition to the parts to compose the diapers 1 on the continuous sheet 3a, and similarly, a plurality of parts to compose the diapers 1 lined in the transporting direction with the product pitch P6 also exist on the pair of fastening tape continuous bodies 6a and 6a in the CD direction.

Here, assume a case of finding the processing device in a position with the greatest number of parts to compose the diapers 1 located between the processing device and the merge position Sc, the number of parts to compose the diapers 1 including the parts in the middle of processing with the processing devices, such as the absorbent bodies 2 in the middle of being generated in the recessed portions 22h of the rotating drum 22, and defining the found processing device as an "uppermost-stream processing device Mu" for convenience. In this example, candidates for the upper-most processing device Mu are the following three processing devices. Specifically, the three processing devices are the adhesive application device 77 in the target-tape transport path R5a, the adhesive application device 67 in the fastening-tape transport path R3a, and the fiber-stacking drum device 21, in Fig. 4.

When counting the number of parts for each of the three devices 77, 67, and 21, in this example, the number for the adhesive application device 77 is 14 (Fig. 5), the number for the adhesive application device 67 is 13 (Fig. 6), and the number for the fiber-stacking drum device 21 is 20 (Fig. 4). Hence, in this example, the fiber-stacking drum device 21 corresponds to the uppermost-stream processing device Mu (Fig. 4).

Meanwhile, as illustrated in Fig. 4, generation of the absorbent bodies 2 is stopped in the middle in the recessed portions 22h and 22h located in the spray duct 23 in the outer circumferential surface of the rotating drum 22 of the fiber-stacking drum device 21. The pulp fiber and SAP of the absorbent bodies 2 in the middle of being generated are parts that can be associated with the diapers 1. In other words, these are parts that can be disposed by finding the number to be disposed in the form of the diapers 1. Hence, the pulp fiber and SAP of the absorbent bodies 2 in the middle of being generated in the uppermost-stream recessed portion 22h among the recessed portions 22h and 22h located in the spray duct 23 correspond to an uppermost-stream part Bue in the area for disposal as a result of corresponding suspension of the transport. Hence, by disposing, as defective pieces, the diapers 1, 1, ... that pass the end cutter device 16 before the uppermost-stream part Bue reaches the position of the end cutter device 16 after the restart of the transport, it is possible to basically prevent the defective pieces from being brought to the market.

However, here, assume a case that, after the restart of the transport, the timing for restoring the devices 77, 52, 53, 55, and 57 in the standby state to the processing state is delayed significantly, and consequently the parts of the back-sheet continuous sheet 5a and the target-tape continuous sheet 7a for which the processing with the devices 77, 52, 53, 55, and 57 is restarted pass the merge position Sc after the uppermost-stream part Bue passes the merge position Sc, the number of defective diapers 1, 1, ... to be disposed increases for the number corresponding to the delay in passing. This may consequently reduce the yields.

In view of this, in the present embodiment, the timing for restoring the devices 77, 52, 53, 55, and 57 in the standby state to the processing state is set as follows. Specifically, the devices 77, 52, 53, 55, and 57 are restored to the processing state such that the parts of the back-sheet continuous sheet 5a and the target-tape continuous sheet 7a for which the processing is restarted after the devices 77, 52, 53, 55, and 57 are restored from the standby state to the processing state pass the merge position Sc before the uppermost-stream part Bue located at the uppermost-stream processing device Mu in the suspension of the transport reaches the merge position Sc after the restart of the transport. Details are as follows.

For example, the adhesive application device 77 in the target-tape transport path R7a is restored to the processing state such that the target tape 7 corresponding to the part for which application of the adhesive is restarted on the target-tape continuous body 7a passes the merge position Sc before the uppermost-stream part Bue reaches the merge position Sc after the restart of the transport.

Similarly, the slip-cutting device 52 in the back-sheet transport path R5a is restored to the processing state such that the part at which handover of the target tape 7 on the back-sheet continuous sheet 5a is restarted passes the merge position Sc before the uppermost-stream part Bue reaches the merge position Sc.

Furthermore, the temporary-pressing device 53 in the back-sheet transport path R5a is restored to the processing state such that the part in which temporary fixing of the target tape 7 on the back-sheet continuous sheet 5a is restarted passes the merge position Sc before the uppermost-stream part Bue reaches the merge position Sc.

Similarly, the main-pressing device 55 in the back-sheet transport path R5a is restored to the processing state such that the part in which fixing of the target tape 7 on the back-sheet continuous sheet 5a is restarted passes the merge position Sc before the uppermost-stream part Bue reaches the merge position Sc.

Furthermore, similarly, the adhesive application device 57 in the back-sheet transport path R5a is restored to the processing state such that the part in which application of the adhesive on the back-sheet continuous sheet 5a is restarted passes the merge position Sc before the uppermost-stream part Bue reaches the merge position Sc. In this way, reduction of the yields is prevented.

Such adjustment of timing of restoration to the processing state may be performed on the basis of the synchronization signal. Specifically, the number of parts to compose the diapers 1 existing between the merge position Sc and each of the devices 77, 52, 53, 55, and 57 is known in advance in association with the corresponding device 77, 52, 53, 55, or 57. Hence, the difference (referred to as a number difference below) between the number of parts to the uppermost-stream part Bue and the number of parts to each of the devices 77, 52, 53, 55, and 57 can be obtained in association with each of the devices 77, 52, 53, 55, and 57, by subtracting the latter number from the former number. Hence, by counting the number of unit signals output in the synchronization signal after the restart of the transport and restoring each of the devices 77, 52, 53, 55, and 57 corresponding to the number difference from the standby state to the processing state before the count value reaches the value of the number difference (e.g., at the time when the count value reaches a value (natural number) obtained by subtracting one from the value of the number difference, or at the time when the count value reaches the value (natural number) obtained by subtracting a value that is two or greater from the value of the number difference), restoration at restoration timing as that described above can be performed. The restoration operation may be performed manually by an operator or may be performed automatically with a control device (not illustrated), such as a computer configured to control the devices 77, 52, 53, ....

A further description will be given of the restoration timing for the plurality of processing devices 77, 52, 53, 55, and 57 to be restored to the processing state after the restart of the transport as described above. It is desirable that, after a processing device located upstream in the transporting direction (corresponding to the first processing device) is restored to the processing state, a processing device (corresponding to the second processing device) located downstream of the processing device in the transporting direction is restored to the processing state.

For example, in the example in Fig. 4 and Fig. 5, the adhesive application device 77 of the target-tape transport path R7a, and the slip-cutting device 52, the temporary-pressing device 53, the main-pressing device 55, and the adhesive application device 57 of the back-sheet transport path R5a lined in this order from the upstream to the downstream in the transporting direction are provided as the plurality of processing devices to which the above-described restoration timing is applied. Hence, in this case, it is preferable that the devices, i.e., the adhesive application device 77, the slip-cutting device 52, the temporary-pressing device 53, the main-pressing device 55, and the adhesive application device 57 are sequentially restored to the processing state, in this order.

In this way, a processing device located downstream, e.g., the main-pressing device 55, can be restored to the processing state in sufficient time compared to a case of restoring the processing device located downstream to the processing state together with a processing device located upstream, e.g., the adhesive application device 77 and the like. Hence, stable restoration of a processing device located downstream such as the main-pressing device 55 to the processing state can be performed.

Assume a case that a processing device located upstream, such as the adhesive application device 77, is restored to the processing state later than a processing device located downstream, such as the main-pressing device 55. In this case, the number of diapers 1 to be defective pieces due to the former processing device may increase. However, by restoring, to the processing state, the processing devices from the one located upstream as described above, this problem can be prevented.

Furthermore, even in a case that a processing device located upstream and a processing device located downstream have a dependency relationship in which a problem occurs if a part processed by the processing device located upstream is not processed by the processing device located downstream, this situation can be managed without any big problem if the processing devices are restored to the processing state from the one located upstream as described above.

In the example in Fig. 5, examples of devices having such a dependency relationship may be the adhesive application device 77 and the temporary-pressing device 53. Specifically, if a portion, of the target-tape continuous body 7a, to which adhesive is not applied is slip-cut to generate the target tape 7 and the temporary-pressing device 53 performs a pressing process, which is a temporary-pressing process, on the target tape 7, the target tape 7 remains in a state of being attached to the back-sheet continuous sheet 5a in an imperfect manner while the adhesive is not applied yet and may be transported in this state integrally with the back-sheet continuous sheet 5a. However, since this target tape 7 is not fixed with so-called adhesive, the target tape 7 is highly likely to come off from the back-sheet continuous sheet 5a in a bent portion in the back-sheet transport path R5a or the like. If the target tape 7 comes off, this is highly likely to cause a trouble, for example, that the target tape 7 is entangled with the various devices 55 and 57 in the transport path R5a. Hence, the adhesive application device 77 and the temporary-pressing device 53 have a dependency relationship to be a cause of a problem.

In a case of having such a dependency relationship, if the temporary-pressing device 53 located downstream is restored to the processing state after the adhesive application device 77 located upstream is restored to the processing state, it is easier for the target tape 7 to be temporarily fixed firmly to the back-sheet continuous sheet 5a with adhesive when the temporary-pressing device 53 performs the pressing process. Hence, occurrence of a problem of coming off described above and the like can be reduced.

Moreover, it is desirable that a control device (not illustrated) such as a computer configured to control operations of the processing devices 77, 52, 53, ... in the manufacturing line LM has an interlock for the order of restoration of the processing devices 77 and 53 having the above-described dependency relationship. In such a case, the interlock is stored in advance in a memory of the control device as one of programs to be used with the control device to control the processing devices 77, 52, 53, .... The control device reads the program of the interlock from the memory and executes the program, to regulate restoration of a processing device located downstream, such as the temporary-pressing device 53, to the processing state before restoration of a processing device located upstream, such as the adhesive application device 77, to the processing state, on the basis of the interlock. For example, in a case of not being in a state where a sensor or the like has detected that the adhesive application device 77 is in the processing state, which is the application state, the control device regulates an operation of the actuator of the temporary-pressing device 53 such that the temporary-pressing device 53 would not be switched to the pressing state, which is the processing state. In this way, it is possible to effectively prevent restoration of a processing device located downstream, such as the temporary-pressing device 53, to the processing state first due to an error, such as a manual operation by an operator.

Note that, for the processing devices not having a dependency relationship as that described above, restoration to the processing state need not be performed from the one located upstream.

For example, the adhesive application device 77 in Fig. 5 and the adhesive application device 57 located downstream of the device 77 in the transporting direction do not have a dependency relationship as that described above. In other words, no problem occurs even if the latter adhesive application device 57 does not apply adhesive to a portion to which the former adhesive application device 77 has applied adhesive. Hence, the latter adhesive application device 57 may apply adhesive prior to the former adhesive application device 77 located upstream.

Meanwhile, in each of the transport paths R3a, R5a, and R1a in the manufacturing line LM in Fig. 4, transport of the corresponding one of the top-sheet continuous sheet 3a, the back-sheet continuous sheet 5a, and the base sheet 1a is performed by applying tension toward the transporting-direction downstream (so-called transport tension) to the corresponding one of the sheets 3a, 5a, and 1a. Hence, the sheets 3a, 5a, and 1a are transported in a state of being stretched in the transporting direction during the production of the diapers 1. Here, the base sheet 1a may desirably be maintained in a state of being stretched in the transporting direction even during the above-described suspension of the production, i.e., even during the suspension of the transport in all the transport paths R1a, R2, R3a, R5a, R6a, and R7a, which is employed in this example.

In the case where the base sheet 1a is maintained in such a stretched state, restart of the transport in the base-sheet transport path R1a, which is a transport path located downstream of the merge position Sc, can be performed smoothly since the base sheet 1a is located downstream of the merge position Sc. Moreover, since each of the sheets 3a, 5a, and 1a is transported with tension toward the downstream as described above in this manufacturing line LM, the transport in the base-sheet transport path R1a located downstream of the merge position Sc may affect the transport of each of the continuous sheets 5a and 3a in the back-sheet transport path R5a and the top-sheet transport path R3a located upstream of the merge position Sc, to no small extent. Hence, smooth restart of the transport in the base-sheet transport path R1a as described above effectively contributes to smooth restart of the transport in each of the back-sheet transport path R5a and the top-sheet transport path R3a, which are transport paths located upstream of the merge position Sc.

The base sheet 1a can be maintained in such a stretched state during suspension of the transport by using suction belt conveyers for the transport devices CV1a and CV1a in the base-sheet transport path R1a in Fig. 7. Specifically, in this example, a suction belt conveyer is provided as the transport device CV1a in a position between the merge position Sc and the leg-hole cutting device 12 in the base-sheet transport path R1a, and a suction belt conveyer is provided as the transport device CV1a in a position between the leg-hole cutting device 12 and the end-cutting device 16 in the transport path R1a. Here, each of the suction belt conveyers includes an endless belt CVlaB driven to rotate in the transporting direction, and a plurality of suction holes (not illustrated) are formed in an outer circumferential surface of the endless belt CV1aB. Thereby, the outer circumferential surface of the endless belt CV1aB is given suction and holding force by suction through the suction holes, and the base sheet 1a is transported by being sucked to the outer circumferential surface with the suction and holding force. In this example, the suction operation with the suction holes is maintained not only during transport but also during suspension of transport. Hence, the base sheet 1a is maintained in a state of being stretched in the transporting direction even during suspension of transport.

In this example, as described above, the leg-hole cutting device 12 (corresponding to a third processing device) and the end-cutting device 16 (corresponding to the third processing device) in the base-sheet transport path R1a are each maintained in the processing state, during the suspension of the transport. Hence, even during the suspension of the transport, the base sheet 1a is basically maintained in a state of being clamped with the pair of upper and lower rolls 12u and 12d and the pairs of upper and lower rolls 16u and 16d of the devices 12 and 16. This also effectively contributes to maintaining the base sheet 1a in a stretched state.

### Other Embodiments

A description has been given of the embodiment of the present invention. The foregoing embodiment is for facilitating the understanding of the present invention and is not to be construed as limiting the present invention. The present invention may be modified and/or improved. For example, the following modifications are possible.

In the above-described embodiment, the tape-type disposable diaper 1 is described as an example of an absorbent article. However, the absorbent article is not limited to this. For example, the absorbent article may be a pull-on disposable diaper (not illustrated). The pull-on disposable diaper may be a so-called three-piece type diaper, in which a liquid-absorptive absorbent main body (a sheet member including the absorbent body 2 inside) bridges a front-side band member and a back-side band member in a spread-out state, or may be a so-called two-piece type diaper, in which an absorbent main body is placed on a skin-side surface of an exterior sheet in a substantially hourglass shape in a spread-out state. Further, the absorbent article is not limited to the above-described disposable diaper 1 and may be any article that absorbs excrement of a wearer. For example, the absorbent article may be a sanitary napkin, a urine-absorbing pad, or the like.

In the above-described embodiment, a description is given by taking a situation in which part of the target-tape continuous body 7a is close to break, as an example of a problem occurring when the transport in each of the transport paths R1a, R2, R3a, R5a, R6, and R7a in the manufacturing line LM is suspended. However, the problem is not limited to this. For example, the above-described "problem" may be a situation in which a part of any of the fastening-tape continuous body 6a, the top-sheet continuous sheet 3a, and the back-sheet continuous sheet 5a is close to break, may be a situation in which any of the continuous body 6a, the sheets 3a and 5a, and the like meanders in the CD direction to a large extent instead of being close to break, or may be other situations than these.

In the above-described embodiment, as illustrated in Fig. 4, all the processing devices 77, 52, 53, 55, and 57 located in the secondary-trouble risk area are brought into the standby state, by setting, as the secondary-trouble risk area, the area from the position St where a problem has occurred, i.e., the position St in which there was a cause of being close to break in the target-tape continuous body 7a, to the merge position Sc. Further, transport in each of the transport paths R1a, R2, R3a, R5a, R6a, and R7a is restarted while the processing devices 77, 52, 53, 55, and 57 are maintained in the standby state. However, the restart is not limited to this. For example, not all but at least some of the processing devices 77, 52, 53, 55, and 57 located in the secondary-trouble risk area may be brought into the standby state, and the transport in each of the transport paths R1a, R2, R3a, R5a, R6a, and R7a may be restarted while some of the processing devices are maintained in the standby state. Note that, in this case, secondary-trouble prevention effect is somewhat reduced but still reasonable prevention effect can be obtained.

In the above-described embodiment, transport in each of the transport paths R1a, R2, R3a, R5a, R6a, and R7 is restarted while only the processing devices 77, 52, 53, 55, and 57 located in the secondary-trouble risk area are maintained in the standby state. However, the restart is not limited to this. For example, transport in each of the transport paths R1a, R2, R3a, R5a, R6a, and R7 may be restarted while some of the processing devices 67, 32, 33, 35, and 37 (corresponding to the fourth processing device) located outside the secondary-trouble risk area are also maintained in the standby state. In this case, the restoration timing for restoring each processing device brought into the standby state to the processing state may be the above-described timing. Specifically, the above processing devices may be restored to the processing state such that the portions for which the processing is restarted after restoration from the standby state to the processing state pass the merge position Sc before the uppermost-stream part Bue located at the uppermost stream processing device Mu in the suspension of the transport reaches the merge position Sc after the restart of the transport.

In the above-described embodiment, as illustrated in Fig. 4, the fiber-stacking drum device 21, the adhesive application devices 67 and 77, the slip-cutting device 32 and 52, the temporary-pressing devices 33 and 53, the main-pressing devices 35 and 55, the adhesive application devices 37 and 57, the leg-hole cutting device 12, the end-cutting device 16, and the like are given as examples of the processing devices. However, the processing devices are not limited to these. Specifically, in the manufacturing line LM, devices other than these may be used, and, for example, a heat-sealing device (not illustrated) may be used. Note that the heat-sealing device is a device configured to form a weld on a processing target, such as the top-sheet continuous sheet 3a, the back-sheet continuous sheet 5a, or the base sheet 1a. Specifically, the device includes a pair of upper and lower rolls each of which is driven with a servomotor to rotate about a rotary driving shaft along the CD direction, to form a weld on the processing target by partially clamping the processing target with a heated protruding portion on an outer circumferential surface of at least one of the pair of rolls and the other roll. Here, also in this heat-sealing device, while the lower roll is rotatably supported in a fixed position, the upper roll is supported with an appropriate actuator, such as a hydraulic cylinder, in such a manner that the length of the gap between the upper roll and the lower roll can be changed, for example. Hence, by controlling the actuator, the device can be switched between the "processing state", which is a state of setting the gap to be small to form the weld, and the "standby state", which is a state of setting the gap to be large not to form the weld.

In the above-described embodiment, as illustrated in Fig. 4, the two continuous sheets 3a and 5a are merged as a plurality of continuous sheets in the manufacturing line LM. Hence, the merge position Sc is a single one, and the merge position Sc corresponds to the "merge completion position" in the claims. However, the merge is not limited to these. Specifically, three or more continuous sheets may be merged in the manufacturing line LM. However, in this case, a plurality of merge positions for continuous sheets may exist in the transporting direction. Here, the "merge completion position" in the claims is a position at which a merge process for all continuous sheets completes, and hence, the merge completion position is a merge position located at the transporting-direction lowermost stream among the plurality of merge positions.

In the above-described embodiment, a case in which the uppermost-stream processing device Mu is the fiber-stacking drum device 21 has been described. However, the uppermost-stream processing device Mu is not limited to this. For example, a different processing device may be the uppermost-stream processing device Mu in some cases depending on the layout or device configuration in the manufacturing line LM. For example, the adhesive application devices 77 or 67 may be the uppermost-stream processing device Mu in some cases, or, in the case that there is another processing device further upstream, the other processing device may become the uppermost-stream processing device Mu in some cases. If either the adhesive application device 77 or the adhesive application device 67 becomes the uppermost-stream processing device Mu, the uppermost-stream part Bue is the uppermost-stream part among parts of the target-tape continuous body 7a and the fastening-tape continuous body 6a to which adhesive has been applied.

In the above-described embodiment, the unit signal of the synchronization signal is described as a signal indicated with a rotational angle value of 0 degrees to 360 degrees. However, the unit signal is not limited to this. For example, the unit signal of the synchronization signal may be configured by a digital value such as 0 to 8191.

### Reference Signs List

1: Disposable diaper (absorbent article);
1LH: Leg opening;
1f: Front-side portion;
1b: Back-side portion;
1p: Part;
1a: Base sheet (composite sheet);
2: Absorbent body;
3: Top sheet;
3a: Top-sheet continuous sheet (component);
3C: Material coil;
5: Back sheet;
5a: Back-sheet continuous sheet (component);
5c: Material coil;
6: Fastening tape;
6a: Fastening-tape continuous body (component);
6ae: Downstream-end portion;
6m: Male material;
7: Target tape;
7a: Target-tape continuous body (component);
7ae: Downstream-end portion;
12: Leg-hole cutting device (processing device, third processing device);
12u: Upper roll;
12uC: Cutter blade;
12d: Lower roll;
16: End-cutting device (processing device, third processing device);
16u: Upper roll;
16uC: Cutter blade;
16d: Lower roll;
21: Fiber-stacking drum device (processing device);
22: Rotating drum;
22h: Recessed portion;
23: Spray duct;
31: Feeding device;
31a: Rotary driving shaft;
32: Slip-cutting device (processing device, fourth processing device);
32A: Anvil roll;
32C: Cutter roll;
32B: Suction blower;
32S: Supply device;
33: Temporary-pressing device (processing device, fourth processing device);
33P: Temporary-pressing roll;
35: Main-pressing device (processing device, fourth processing device);
35P1: Roll;
35P2: Roll;
37: Adhesive application device (processing device, fourth processing device);
37N: Nozzle;
51: Feeding device;
51a: Rotary driving shaft;
52: Slip-cutting device (processing device);
52A: Anvil roll;
52AC: Receiving blade;
52C: Cutter roll;
52CC: Cutter blade;
52B: Suction blower;
52BH: Suction port;
52S: Supply device;
53: Temporary-pressing device (processing device, second processing device);
53P: Temporary-pressing roll;
55: Main-pressing device (processing device);
55P1: Roll;
55P2: Roll;
57: Adhesive application device (processing device);
57N: Nozzle;
67: Adhesive application device (processing device, fourth processing device);
67N: Nozzle;
77: Adhesive application device (processing device, first processing device);
77N: Nozzle;
LM: Manufacturing line;
Mu: Uppermost-stream processing device;
B: Component;
Bue: Uppermost-stream part;
R1a: Base-sheet transport path (transport path);
R2: Absorbent-body transport path (transport path);
R3a: Top-sheet transport path (transport path);
R5a: Back-sheet transport path (transport path);
R6a: Target-tape transport path (transport path);
R7a: Fastening-tape transport path (transport path);
R22: Transport path;
Sc: Merge position (merge completion position);
St: Position where problem has occurred (certain position);
S6: Predetermined position;
S7: Predetermined position;
S12: Predetermined position;
S16: Predetermined position;
Sout: Handover position;
CV1a: Transport device;
CV1aB: Endless belt;
CV2: Belt conveyer;
G12: Gap;
G16: Gap;
G35P: Gap;
G55P: Gap.

## Claims

1. A method for preventing an increase in the number of defective absorbent articles in a method for manufacturing the absorbent article by processing a plurality of components including a plurality of continuous sheets while transporting the plurality of components in a plurality of transport paths, the method comprising:
a generation step of generating a single composite sheet that is continuous in a transporting direction by completing in a predetermined merge completion position a merge process of all the continuous sheets of the plurality of continuous sheets;
a generation step of generating the absorbent article by cutting the composite sheet at an interval in the transporting direction;
a transport suspension step of suspending transport in the plurality of transport paths at an occurrence of a problem in a certain position in the plurality of transport paths;
a standby step of bringing into a standby state at least some of processing devices among a plurality of processing devices located between the certain position where the problem has occurred and the merge completion position;
a transport restart step of restarting the transport, after correcting the problem, while some of the processing devices are maintained in the standby state; and
a restoration step of restoring to a processing state some of the processing devices in the standby state, after restarting the transport, wherein
in a case of defining, as an uppermost-stream processing device as viewed in the transport direction, a processing device in a position with a greatest number of parts to compose the absorbent article, among all processing devices located upstream of the merge completion position in the transporting direction, the parts being placed between a position of each processing device and the merge completion position and including the parts in the middle of being processed with the processing device,
the restoration step is performed such that a part for which processing is restarted in the restoration step passes the merge completion position before an uppermost-stream part, as viewed in the transport direction, reaches the merge completion position after the restart of the transport, the uppermost-stream part being a part that can be associated with the absorbent article among parts of the plurality of components, the uppermost-stream part being located in the uppermost-stream processing device during suspension of the transport.

2. The method according to claim 1, wherein
some of the processing devices in the standby state include a first processing device and a second processing device located downstream of the first processing device in the transporting direction, and
the second processing device is restored to the processing state after the first processing device is restored to the processing state.

3. The method according to claim 2, wherein
a dependency relationship in which a problem occurs if the second processing device does not process a part processed with the first processing device exists between the first processing device and the second processing device.

4. The method according to claim 2 or 3, wherein
a control device configured to control the first processing device and the second processing device is provided, and
the control device includes an interlock for regulating a situation that the second processing device is restored to the processing state before the first processing device is restored to the processing state.

5. The method according to any one of claims 1 to 4, wherein
the composite sheet is transported on a downstream side of the merge completion position in the transporting direction while the composite sheet is in a state of being stretched in the transporting direction, and
the composite sheet is maintained in the state of being stretched in the transporting direction even during the suspension of the transport in the plurality of transport paths.

6. The method according to claim 5, wherein
a conveyer configured to transport the composite sheet is provided,
the conveyer is a suction belt conveyer with a suction-holding function in an outer circumferential surface of an endless belt, and
the composite sheet is maintained in the state of being stretched in the transporting direction even during the suspension of the transport by being sucked and held to the outer circumferential surface of the endless belt.

7. The method according to claim 5 or 6, wherein
a third processing device configured to process the composite sheet is provided in a transport path for transporting the composite sheet,
the third processing device includes a pair of rolls configured to rotate in a transporting direction of the composite sheet, the pair of rolls being configured to be switchable between a processing state with a small gap between the rolls and a standby state with a greater gap than the gap in the processing state,
in the processing state, the pair of rolls clamps the composite sheet to process the composite sheet, and
the third processing device is maintained in the processing state even during the suspension of the transport in the plurality of transport paths.

8. The method according to claim 7, wherein
a fourth processing device located in a position outside an area from the certain position where the problem has occurred to the merge completion position is provided,
the fourth processing device is in a standby state at a time of the restart of the transport, and
the fourth processing device is restored to the processing state such that a part for which processing is restarted after the fourth processing device is restored from the standby state to the processing state passes the merge completion position, before the uppermost-stream part reaches the merge completion position, after the restart of the transport.

## Patentansprüche

1. Verfahren zum Verhindern einer Zunahme in der Anzahl von fehlerhaften absorbierenden Artikeln in einem Verfahren zur Herstellung des absorbierenden Artikels durch Verarbeiten einer Vielzahl von Komponenten, die eine Vielzahl von kontinuierlichen Lagen umfassen, während die Vielzahl von Komponenten in einer Vielzahl von Transportwegen transportiert wird, wobei das Verfahren umfasst:
einen Erzeugungsschritt des Erzeugens einer einzelnen Verbundlage, die in einer Transportrichtung kontinuierlich ist, indem in einer vorbestimmten Zusammenführungsabschlussposition ein Zusammenführungsprozess aller kontinuierlichen Lagen der Vielzahl von kontinuierlichen Lagen abgeschlossen wird;
einen Erzeugungsschritt des Erzeugens des absorbierenden Artikels durch Schneiden der Verbundlage in einem Intervall in der Transportrichtung;
einen Transportunterbrechungsschritt des Unterbrechens des Transports in der Vielzahl von Transportwegen bei einem Auftreten eines Problems in einer bestimmten Position in der Vielzahl von Transportwegen;
einen Bereitschaftsschritt des in einen Bereitschaftszustand Bringens mindestens einiger Verarbeitungsvorrichtungen aus einer Vielzahl von Verarbeitungsvorrichtungen, die zwischen der bestimmten Position, an der das Problem aufgetreten ist, und der Zusammenführungsabschlussposition angeordnet sind;
einen Transportwiederaufnahmeschritt zum Wiederaufnehmen des Transports nach Behebung des Problems, während einige der Verarbeitungsvorrichtungen in dem Bereitschaftszustand gehalten werden; und
einen Wiederherstellungsschritt des in einen Verarbeitungszustand Wiederherstellens einiger der Verarbeitungsvorrichtungen in den Bereitschaftszustand nach dem Neustart des Transports, wobei
in einem Fall des Definierens, als in der Transportrichtung betrachtet, einer vorgelagertsten Verarbeitungsvorrichtung, einer Verarbeitungsvorrichtung in einer Position mit einer größten Anzahl von Teilen, um den absorbierenden Artikel zusammenzusetzen, unter allen Verarbeitungsvorrichtungen, die in der Transportrichtung der Zusammenführungsabschlussposition vorgelagert angeordnet sind, wobei die Teile zwischen einer Position jeder Verarbeitungsvorrichtung und der Zusammenführungsabschlussposition angeordnet sind und die Teile in der Mitte der Verarbeitung mit der Verarbeitungsvorrichtung umfassen,
wobei der Wiederherstellungsschritt derart ausgeführt wird, dass ein Teil, für das die Verarbeitung im Wiederherstellungsschritt wieder aufgenommen wird, die Zusammenführungsabschlussposition passiert, bevor ein in der Transportrichtung gesehen vorgelagertstes Teil nach dem Neustart des Transports die Zusammenführungsabschlussposition erreicht, wobei das vorgelagertste Teil ein Teil ist, das dem absorbierenden Artikel unter Teilen der Vielzahl von Komponenten zugeordnet werden kann, wobei das vorgelagertste Teil während der Unterbrechung des Transports in der vorgelagertsten Verarbeitungsvorrichtung angeordnet ist.

2. Verfahren zur Herstellung eines absorbierenden Artikels gemäß Anspruch 1, wobei
einige der Verarbeitungsvorrichtungen in dem Bereitschaftszustand eine erste Verarbeitungsvorrichtung und eine zweite Verarbeitungsvorrichtung umfassen, die in der Transportrichtung hinter der ersten Verarbeitungsvorrichtung angeordnet ist, und
die zweite Verarbeitungsvorrichtung in den Verarbeitungszustand zurückversetzt wird, nachdem die erste Verarbeitungsvorrichtung in den Verarbeitungszustand zurückversetzt wurde.

3. Verfahren zur Herstellung eines absorbierenden Artikels gemäß Anspruch 2, wobei
eine Abhängigkeitsbeziehung, in der ein Problem auftritt, wenn die zweite Verarbeitungsvorrichtung ein mit der ersten Verarbeitungsvorrichtung verarbeitetes Teil nicht verarbeitet, zwischen der ersten Verarbeitungsvorrichtung und der zweiten Verarbeitungsvorrichtung besteht.

4. Verfahren zur Herstellung eines absorbierenden Artikels gemäß Anspruch 2 oder 3, wobei
eine Steuervorrichtung zum Steuern der ersten Verarbeitungsvorrichtung und der zweiten Verarbeitungsvorrichtung vorgesehen ist, und
die Steuervorrichtung eine Verriegelung zur Regelung einer Situation umfasst, in der die zweite Verarbeitungsvorrichtung in den Verarbeitungszustand zurückversetzt wird, bevor die erste Verarbeitungsvorrichtung in den Verarbeitungszustand zurückversetzt wird.

5. Verfahren zur Herstellung eines absorbierenden Artikels gemäß einem der Ansprüche 1 bis 4, wobei
die Verbundlage auf einer nachgelagerten Seite der Zusammenführungsabschlussposition in der Transportrichtung transportiert wird, während die Verbundlage in der Transportrichtung gedehnt wird, und
die Verbundlage auch während der Unterbrechung des Transports in der Vielzahl der Transportwege in dem in der Transportrichtung gedehnten Zustand gehalten wird.

6. Verfahren zur Herstellung eines absorbierenden Artikels gemäß Anspruch 5, wobei
eine Fördervorrichtung, der für den Transport der Verbundlage ausgebildet ist,
wobei die Fördervorrichtung ein Saugbandförderer mit einer Saug-Haltefunktion in einer äußeren Umfangsoberfläche eines Endlosbandes ist, und
wobei die Verbundlage auch während der Unterbrechung des Transportes durch Ansaugen und Halten an der äußeren Umfangsoberfläche des Endlosbandes in dem in der Transportrichtung gedehnten Zustand gehalten wird.

7. Verfahren zur Herstellung eines absorbierenden Artikels gemäß Anspruch 5 oder 6, wobei
eine dritte Verarbeitungsvorrichtung, die für die Verarbeitung der Verbundlage ausgebildet ist, in einem Transportweg für den Transport der Verbundlage vorgesehen ist,
wobei die dritte Verarbeitungsvorrichtung ein Walzenpaar umfasst, das zum Drehen in einer Transportrichtung der Verbundlage ausgebildet ist, wobei das Walzenpaar ausgebildet ist zwischen einem Verarbeitungszustand mit einem kleinen Spalt zwischen den Walzen und einem Bereitschaftszustand mit einem größeren Spalt als dem Spalt im Verarbeitungszustand umschaltbar zu sein,
wobei in dem Verarbeitungszustand, das Walzenpaar die Verbundlage einklemmt, um die Verbundlage zu verarbeiten, und
wobei die dritte Verarbeitungsvorrichtung auch während der Unterbrechung des Transports in der Vielzahl der Transportwege in dem Verarbeitungszustand gehalten wird.

8. Verfahren zur Herstellung eines absorbierenden Artikels gemäß Anspruch 7, wobei
eine vierte Verarbeitungsvorrichtung, die an einer Position außerhalb eines Bereiches von der bestimmten Position, an der das Problem aufgetreten ist, bis zu der Zusammenführungsabschlussposition angeordnet ist,
wobei die vierte Verarbeitungsvorrichtung sich zu einem Zeitpunkt der Wiederaufnahme des Transports in einem Bereitschaftszustand befindet, und
wobei die vierte Verarbeitungsvorrichtung in den Verarbeitungszustand zurückversetzt wird, so dass ein Teil, für das die Verarbeitung wieder aufgenommen wird, nachdem die vierte Verarbeitungsvorrichtung aus dem Bereitschaftszustand in den Verarbeitungszustand zurückversetzt wurde, nach der Wiederaufnahme des Transports die Zusammenführungsabschlussposition passiert, bevor das vorgelagertste Teil die Zusammenführungsabschlussposition erreicht.

## Revendications

1. Procédé pour empêcher une augmentation du nombre d'articles absorbants défectueux dans un procédé de fabrication de l'article absorbant en traitant une pluralité de composants comportant une pluralité de feuilles continues tout en transportant la pluralité de composants dans une pluralité de chemins de transport, le procédé comprenant :
une étape de génération consistant à générer une feuille composite unique qui est continue dans une direction de transport en achevant dans une position de fin de fusion prédéterminée un processus de fusion de toutes les feuilles continues de la pluralité de feuilles continues ;
une étape de génération consistant à générer l'article absorbant en coupant la feuille composite à un intervalle dans la direction de transport ;
une étape de suspension de transport consistant à suspendre le transport dans la pluralité de chemins de transport lors de l'apparition d'un problème dans une certaine position dans la pluralité de chemins de transport ;
une étape d'attente consistant à mettre dans un état d'attente au moins certains des dispositifs de traitement parmi une pluralité de dispositifs de traitement situés entre la certaine position où le problème est apparu et la position de fin de fusion ;
une étape de redémarrage de transport pour redémarrer le transport, après avoir corrigé le problème, tandis que certains des dispositifs de traitement sont maintenus dans l'état d'attente ; et
une étape de restauration consistant à restaurer à un état de traitement certains des dispositifs de traitement dans l'état d'attente, après avoir redémarré le transport, dans lequel
en cas de définition, en tant que dispositif de traitement de flux supérieur tel qu'observé dans la direction de transport, d'un dispositif de traitement dans une position avec un plus grand nombre de parties pour composer l'article absorbant, parmi tous les dispositifs de traitement situés en amont de la position de fin de fusion dans la direction de transport, les parties étant placées entre une position de chaque dispositif de traitement et la position de fin de fusion et comportant les parties en cours de traitement avec le dispositif de traitement,
l'étape de restauration est effectuée de sorte qu'une partie pour laquelle le traitement est redémarré dans l'étape de restauration passe la position de fin de fusion avant qu'une partie de flux supérieur, telle qu'observée dans la direction de transport, atteint la position de fin de fusion après le redémarrage du transport, la partie de flux supérieur étant une partie qui peut être associée à l'article absorbant parmi les parties de la pluralité de composants, la partie de flux supérieur étant située dans le dispositif de traitement de flux supérieur pendant la suspension du transport.

2. Procédé selon la revendication 1, dans lequel
certains des dispositifs de traitement à l'état d'attente comportent un premier dispositif de traitement et un deuxième dispositif de traitement situé en aval du premier dispositif de traitement dans la direction de transport, et
le deuxième dispositif de traitement est restauré à l'état de traitement après que le premier dispositif de traitement est restauré à l'état de traitement.

3. Procédé selon la revendication 2, dans lequel
une relation de dépendance dans laquelle un problème apparaît si le deuxième dispositif de traitement ne traite pas une partie traitée avec le premier dispositif de traitement existe entre le premier dispositif de traitement et le deuxième dispositif de traitement.

4. Procédé selon la revendication 2 ou 3, dans lequel
un dispositif de commande configuré pour commander le premier dispositif de traitement et le deuxième dispositif de traitement est prévu, et
le dispositif de commande comporte un verrouillage pour réguler une situation dans laquelle le deuxième dispositif de traitement est restauré à l'état de traitement avant que le premier dispositif de traitement ne soit restauré à l'état de traitement.

5. Procédé selon l'une quelconque des revendications 1 à 4, dans lequel
la feuille composite est transportée sur un côté aval de la position de fin de fusion dans la direction de transport tandis que la feuille composite est dans un état d'étirage dans la direction de transport, et
la feuille composite est maintenue dans l'état d'étirage dans la direction de transport même pendant la suspension du transport dans la pluralité de chemins de transport.

6. Procédé selon la revendication 5, dans lequel
un transporteur configuré pour transporter la feuille composite est prévu,
le transporteur est un transporteur à bande aspirante avec une fonction de maintien d'aspiration dans une surface circonférentielle extérieure d'une bande sans fin, et
la feuille composite est maintenue dans l'état d'étirage dans la direction de transport même pendant la suspension du transport en étant aspirée et maintenue sur la surface circonférentielle extérieure de la bande sans fin.

7. Procédé selon la revendication 5 ou 6, dans lequel
un troisième dispositif de traitement configuré pour traiter la feuille composite est prévu dans un chemin de transport pour transporter la feuille composite,
le troisième dispositif de traitement comporte une paire de rouleaux configurés pour tourner dans une direction de transport de la feuille composite, la paire de rouleaux étant configurée pour être commutable entre un état de traitement avec un petit espace entre les rouleaux et un état d'attente avec un espace plus grand que l'espace dans l'état de traitement,
dans l'état de traitement, la paire de rouleaux serre la feuille composite pour traiter la feuille composite, et
le troisième dispositif de traitement est maintenu dans l'état de traitement même pendant la suspension du transport dans la pluralité de chemins de transport.

8. Procédé selon la revendication 7, dans lequel
un quatrième dispositif de traitement situé dans une position à l'extérieur d'une zone depuis la certaine position où le problème est apparu jusqu'à la position de fin de fusion est prévu,
le quatrième dispositif de traitement est dans un état d'attente au moment du redémarrage du transport, et
le quatrième dispositif de traitement est restauré à l'état de traitement de sorte qu'une partie pour laquelle le traitement est redémarré après que le quatrième dispositif de traitement est restauré de l'état d'attente à l'état de traitement passe la position de fin de fusion, avant que la partie de flux supérieur n'atteigne la position de fin de fusion, après le redémarrage du transport.
